# EUROPEAN PATENT APPLICATION

(11) **EP 4 487 866 A1**
(43) Date of publication of application: **08.01.2025**
(21) Application number: 23189816.4
(22) Date of filing: 04.08.2023
(51) Int. Cl.: A61K 38/18, A61K 31/00, A61K 35/17, A61P 29/00, A61P 31/12, C07K 16/22

(54) **TGF-SS INHIBITION AND ENGINEERED T-CELLS FOR THE TREATMENT OF COVID-19 SEQUELAE AND VIRUS-INDUCED HYPERINFLAMMATION**

(30) Priority: 07.07.2023 EP 23184044
(71) Applicant: Deutsches Rheuma-Forschungszentrum Berlin, 10117 Berlin (DE)
(72) Inventor: Mashreghi, Mir-Farzin, 13089 Berlin (DE); Goetzke, Carl-Christoph, 10115 Berlin (DE); Kallinich, Tilmann, 10435 Berlin (DE); Durek, Pawel, 10557 Berlin (DE)
(74) Representative: Hertin und Partner Rechts- und Patentanwälte PartG mbB

(57) **Abstract**

The invention relates in one aspect to a TGF-β inhibitor for use in the treatment of virus-induced hyperinflammation or (post-)COVID-19 infection symptoms or sequelae, wherein the patient is suffering from or experiencing elevated (above-normal) expression, excretion and/or blood levels of transforming growth factor-β (TGF-β) and/or interferon. The invention relates also to a TGF-β inhibitor for use in the treatment of COVID-19-induced hyperinflammation or (post-)COVID-19 symptoms or sequelae or Long-COVID-19. In another aspect the invention relates to a method for stimulating T cells and to a T-cell for the use in the treatment of virus reactivation and/or SARS-CoV-2-induced hyperinflammation.

## Description

The invention lies in the field of biochemistry and medicine, particularly in the field of therapies of symptoms or sequelae of viral infections and virus-induced hyperinflammation, such as (post-)COVID-19 symptoms or sequelae.

The present invention is directed in one aspect to the targeted intervention involving transforming growth factor-β (TGF-β) blockade for the management of virus-induced hyperinflammation, MIS-C and also other inflammatory (post-)COVID-19 symptoms or sequelae, also known as LONG-COVID-19. In embodiments beyond (post-)COVID-19 sequelae, therapies comprising the administration of autologous Epstein-Barr virus (EBV) or other Herpesviridae (HHV1-8)-specific T-cells engineered to be non-sensitive to TGF-β or immunosuppression can alleviate undesirable virus-induced hyperinflammation.

### BACKGROUND OF THE INVENTION

At the beginning of the COVID-19 pandemic, children appeared to be only mildly affected by SARS-CoV-2 infections (Zhu, Wang et al. 2020). However, in April 2020, British paediatric intensive care physicians observed a cluster of patients with hyperinflammatory shock linked to a previous infection with SARS-CoV-2 (Riphagen, Gomez et al. 2020). Simultaneously, paediatricians in Bergamo documented a significant rise in cases of Kawasaki-like disease (Verdoni, Mazza et al. 2020). These patients had symptoms resembling both a toxic shock syndrome and Kawasaki-shock-syndrome, which usually started 4-8 weeks after infection with SARS-CoV-2 (Belot and Levy-Bruhl 2020). Untreated, this hyperinflammation involving multiple organs led to organ failure (Loomba, Villarreal et al. 2020). In a subset of children and adolescents, SARS-CoV-2 infection induces a severe acute hyperinflammatory shock (Riphagen, Gomez et al. 2020) termed multisystem inflammatory syndrome in children (MIS-C) within 4-8 weeks post-infection. MIS-C is characterised by oligoclonal T-cell expansion (Moreews, Le Gouge et al. 2021) and systemic hyperinflammation (Sacco, Castagnoli et al. 2022). The pathogenesis of multisystem inflammatory syndrome (MIS) and MIS-C remains elusive. Several studies were conducted, testing different hypotheses for the pathogenesis of MIS-C; despite these studies, the pathogenesis of MIS-C remains ill characterised. Some results point to impaired viral clearance and intestinal barrier dysfunction (Yonker, Gilboa et al. 2021), yet in animal models, fever alone can induce similar findings (Lambert 2004). Additionally, while autoantibody formation (Porritt, Binek et al. 2021, Pfeifer, Thurner et al. 2022) has been described in some cases, specificity of auto-antibodies has not consistently been confirmed across different studies. For example, in one cohort, interleukin-1 receptor antagonist (IL-1 Ra) neutralising antibodies were found in 13 of 21 patients. This was accompanied by low levels of IL-1Ra (Pfeifer, Thurner et al. 2022), which could explain the widespread inflammation in these individuals. Others have raised the hypothesis of a superantigen-like immune reaction, as MIS-C is linked to a unique oligoclonal T-cell expansion, and/or activation associated with Vβ21.3 TCR+ CD4 and CD8 T-cells (Cheng, Zhang et al. 2020, Hoste, Roels et al. 2021, Moreews, Le Gouge et al. 2021, Porritt, Paschold et al. 2021). As of now, however, only structural models predict a superantigen-like region within the spike-protein, which could bind to Vβ21.3 TCR (Porritt, Paschold et al. 2021). More recently, autosomal recessive deficiencies in the OAS-RNAse L pathway were found in nearly 1% of affected children, highlighting the importance of monocyte activation in the development of MIS-C (Lee, Le Pen et al. 2023). Taken together, none of these hypotheses can explain the full set of disease features in all cases of MIS and MIS-C.

In light of the prior art there remains a significant need to provide novel means for the management of MIS-C, other inflammatory (post-)COVID-19 sequelae, including Long-COVID-19, as well as undesirable virus-induced hyperinflammation and/or (herpes) virus reactivation, e.g., after COVID-19 infection.

### SUMMARY OF THE INVENTION

The afore problem is solved by the features of the independent claims. Preferred embodiments of the present invention are provided by the dependent claims.

The invention relates in one aspect to a TGF-β inhibitor for use in the treatment of virus-induced hyperinflammation or (post-)infection symptoms or sequelae after viral infection, wherein the patient is suffering from or experiencing elevated (above-normal) expression, excretion and/or blood levels (concentration in the blood) of transforming growth factor-β (TGF-β).

In certain embodiments of the present invention the patient is suffering from or experiencing elevated (above-normal) expression, excretion and/or blood levels (concentration in the blood) of transforming growth factor-β (TGF-β) and interferon.

The invention relates in one embodiment to a TGF-β inhibitor for use in the treatment of COVID-19-induced hyperinflammation or (post-)COVID-19 symptoms or sequelae or Long-COVID-19, wherein the patient is preferably suffering from/experiencing elevated (above-normal/-healthy) expression, excretion and/or blood levels (concentration in the blood) of (active) transforming growth factor-β (TGF-β; TGF-β 1/2/3).

In other words, the invention relates in one aspect to the inhibition of TGF-β expression, excretion and/or the reduction of transforming growth factor-β (TGF-β) blood levels (TGF-β blockade) for treating COVID-19-induced hyperinflammation or (post-)COVID-19 (Long-COVID-19) symptoms or sequelae, wherein the patient is preferably suffering from/experiencing elevated (above-normal/-healthy) expression, excretion and/or blood levels of TGF-β.

Previously the inventors could show that TGF-β serum levels are an early indicator, with high accuracy, that predicts the COVID-19 disease severity, and fatality of thisCOVID-19 patients (Frischbutter et al., 2023). In this previous study the inventors found that patients with severe COVID-19 had significantly higher TGF-β levels compared to patients with mild or moderate COVID-19.

In a subset of children and adolescents, SARS-CoV-2 infection induces a severe acute hyperinflammatory shock (Riphagen, Gomez et al. 2020) termed multisystem inflammatory syndrome in children (MIS-C) within 4-8 weeks post-infection. MIS-C is characterised by oligoclonal T-cell expansion (Moreews, Le Gouge et al. 2021) and systemic hyperinflammation (Sacco, Castagnoli et al. 2022). The pathogenesis of MIS-C remained elusive so far. The inventors surprisingly found that acute MIS-C is characterised by impaired reactivation of virus-reactive memory T-cells, which depends on elevated serum levels of the cytokine TGF-β, levels of which are comparable to severe COVID-19 (Witkowski, Tizian et al. 2021). This functional impairment in T-cell reactivity is accompanied by the presence of TGF-β-response signatures in T-cells, B-cells, and monocytes, along with reduced antigen-presentation capabilities of monocytes and can be reversed by blocking TGF-β. Furthermore, T-cell receptor (TCR) repertoires of MIS-C-patients exhibit expansion of T-cells expressing the Vβ21.3 TCR, resembling Epstein-Barr Virus (EBV)-reactive T-cells clones capable of eliminating EBV-infected B-cells. Clinically, the TGF-β-induced defect in T-cell reactivity correlates with a higher EBV seroprevalence in MIS-C patients compared to age-matched controls, along with the occurrence of EBV reactivation. Additionally, serum TGF-β levels in MIS-C patients can trigger EBV reactivation, which is reversible with TGF-β blockade. The present findings establish a connection between SARS-CoV-2 infection and COVID-19 sequelae in adults and children, where T-cell dysfunction induced by SARS-CoV-2-triggered TGF-β overproduction leads to EBV-reactivation and subsequent hyperinflammation.

Therefore in embodiments the present invention relates to a TGF-β inhibitor for use in the treatment of virus-induced hyperinflammation or (post-) COVID-19 symptoms or sequelae, wherein the patient is suffering from or experiencing elevated (above-normal) expression, excretion and/or blood levels of transforming growth factor-β (TGF-β), and wherein preferably the virus-induced hyperinflammation is associated with, or induced by a COVID-19 infection and/or (dormant) virus, preferably herpes virus, reactivation in the patient.

The inventors herein propose novel treatments and a mechanism of severe acute hyperinflammatory shock, multisystem inflammatory syndrome and chronic inflammation, such as MIS and MIS-C pathogenesis, comprising a combination of genetic susceptibility and reactivation of dormant latent virus infection (frequently EBV) that provides new data for the urgently required treatment for COVID-19-induced hyperinflammation, such as MIS or MIS-C. In individuals with this combined susceptibility, an infection with SARS-CoV-2 triggers latent virus reactivation and induces a prolonged and excessive TGF-β response. The inventors herein show that high TGF-β levels lead to a continued EBV reactivation and reduced functionality of antigen specific T-cells. The resulting viremia leads to oligoclonal expansion of the dysfunctional T cells (TCs), which are incapable of controlling the virus, resulting in a sepsis-like hyperinflammation.

In embodiments the COVID-19 induced hyperinflammation is a severe acute hyperinflammatory shock, a multisystem inflammatory syndrome or a chronic inflammation.

In embodiments the COVID-19-induced hyperinflammation is associated with or induced by a COVID-19 infection. In embodiments the COVID-19-induced hyperinflammation is considered a sequelae of COVID-19 (post COVID-19), also known as Long-COVID-19, and/or is associated with a prior COVID-19 infection, preferably a past or cured infection. In embodiments the COVID-19-induced hyperinflammation is diagnosed or incurs at least 1 week, preferably after 4-8 weeks, after the COVID-19 infection/post-infection.

In embodiments the COVID-19 induced hyperinflammation is a severe acute hyperinflammatory shock or a multisystem inflammatory syndrome (MIS). In embodiments the COVID-19 induced hyperinflammation is a severe acute hyperinflammatory shock or a multisystem inflammatory syndrome in children (MIS-C).

In embodiments the patient suffering from COVID-19 induced hyperinflammation is an adult or a child. In embodiments the patient suffering from COVID-19 induced hyperinflammation is a child, preferably wherein the patient is younger than 21 years, more preferably younger than 16 years.

Herein the inventors have unrevealed through a comprehensive study the mechanistic link between SARS-CoV-2 infection and a post-acute COVID-19 sequelae, particularly in children (Multisystem Inflammatory Syndrome, MIS-C). The inventors revealed that in multisystem inflammatory syndrome, particularly in MIS-C, unlike other measured cytokines and chemokines, TGF-β was significantly induced in all patients, leading to functional impairment of virus-reactive T-cells.

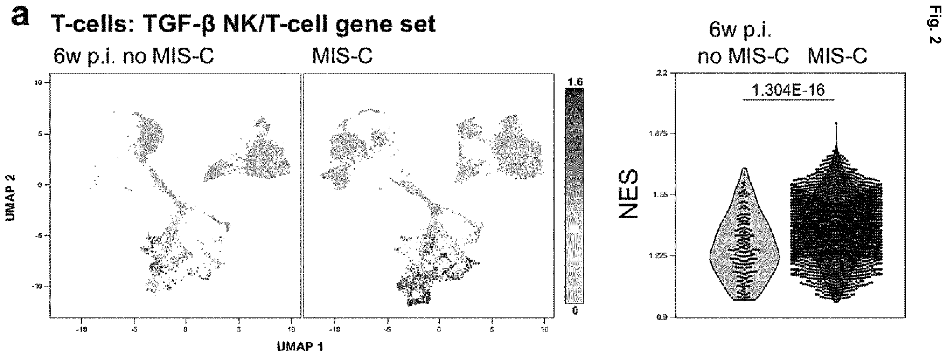

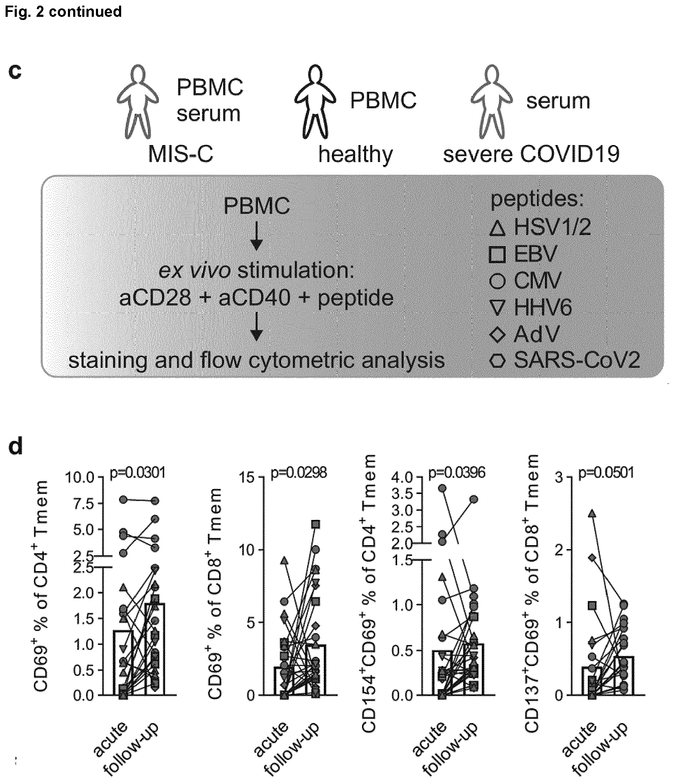

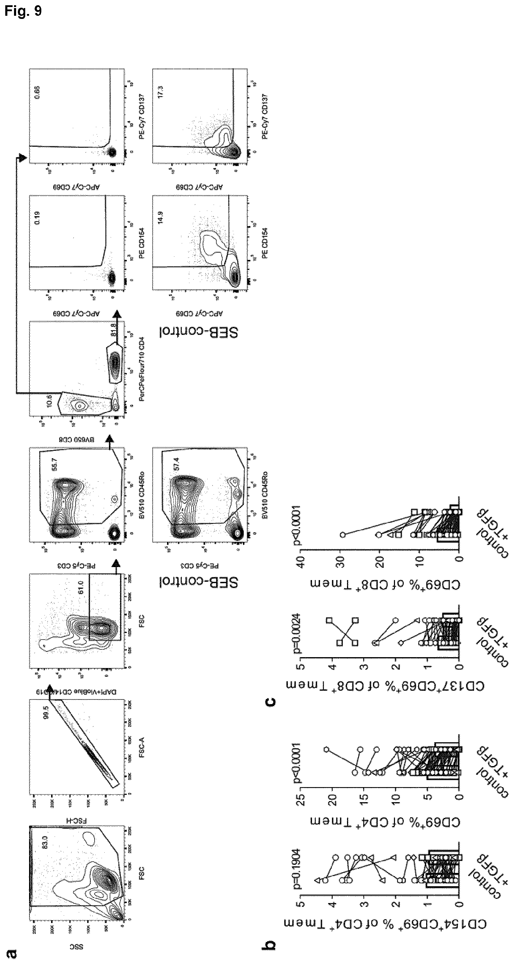

Some aspects of the invention are based on the surprising finding of the inventors, that hyperinflammation in patients (e.g., MIS-C induced by previous viral infections) affects memory T-cell function. The inventors surprisingly found, that during acute MIS-C, both CD4+ and CD8+ T-cells co-cultured with antigen presenting cells and various viral peptides showed functional impairment by reduced expression of the early activation marker CD69 (see e.g., Fig. 2d). Even specific reactivation by these viral peptides was impaired during the acute phase of MIS-C (see Examples). Given the strong TGF-β instruction (stimulation) of T-cells in MIS-C (e.g., see Fig. 2a) and because TGF-β has been implicated in terminating immune responses before, the inventors hypothesized that the observed impairment in memory T-cell reactivation could be reproduced with T-cells derived from healthy donors when stimulated in the presence of TGF-β. Indeed, the inventors reviled that the addition of TGF-β significantly impaired T-cell reactivity (e.g., see Fig. 9b-c). In summary the inventors showed that observed impairment of memory T-cell reactivation could be effectively reversed by blocking TGF-β.

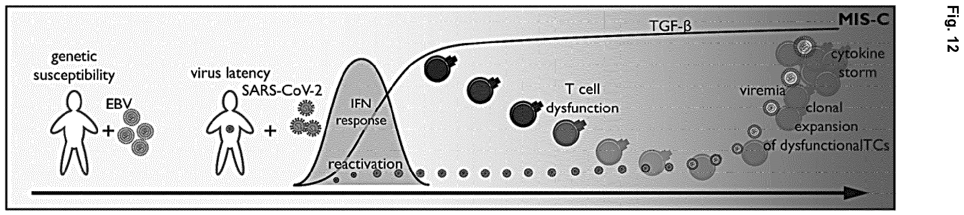

Herein the inventors further showed that in multisystem inflammatory syndrome, particularly in MIS-C, unlike other measured cytokines and chemokines, TGF-β was significantly induced in all patients, leading to functional impairment of virus-reactive T-cells and compromising immune-surveillance of Epstein-Barr virus (EBV). The inventors could show that the uncontrolled presence of EBV in the bloodstream contributes to the observed hyperinflammation in MIS, particularly MIS-C, patients following SARS-CoV-2 infection (see Figure 12).

While several studies have documented the concurrent occurrence of Herpesviridae and (post-) COVID-19 sequelae, the mechanistic connection between them has yet to be established. The inventors previous work has shed light on the role of SARS-CoV-2-induced TGF-β in chronic inflammation (Witkowski, Tizian et al. 2021) and compromising NK-cell function and SARS-CoV-2 clearance in severe COVID-19. Now, the inventors unveiled how this mechanism facilitates the reactivation of dormant latent Herpesviridae, specifically EBV, which employs TGF-β (Liang, Chen et al. 2002) as a means to evade the immune system. Prior studies identified a cohort of children with hyperinflammation exhibiting all clinical and immunological characteristics of MIS-C, despite no prior exposure to SARS-CoV-2. Notably, these patients developed MIS-C before the start of the COVID-19 pandemic. Among them, all tested individuals were seropositive for EBV and in 75% of the patient's reactivation of Herpesviridae, in particular EBV, could be identified by PCR analysis. Furthermore, the same patients had expansion of the TCRVβ21.3+ T-cells, which the inventors show herein to clonally resemble EBV-specific cytotoxic T cells capable of eliminating EBV transformed primary B cells.

The mechanistic insights the inventors present herein regarding the role of TGF-β in MIS, particularly MIS-C, development hold significant implications for the development of targeted therapeutic strategies for affected children and adolescents. The inventor's findings provide evidence for the use of TGF-β blockade in managing MIS-C and other inflammatory (post) COVID-19 sequelae, including Long-COVID-19. Furthermore, the present innovative approach, combining Antigen-reactive T-cell enrichment (ARTE) with HLA-haplotyping to identify highly functional EBV-specific cells, offers new avenues for targeted therapies beyond COVID-19 sequelae. By leveraging T-cell engineering to render these cells insensitive to immunosuppression or TGF-β, the present invention provides methods and means with high efficacies in treating prolonged and/or overwhelming virus-induced inflammation or post-transplant lymphoproliferative disorders.

In another aspect the present invention relates to a TGF-β inhibitor for use in the treatment of reactivation of viruses in a patient, such as herpes viruses (Herpesviridae), wherein the patient is preferably suffering from/experiencing elevated (above-normal/above-healthy) expression, excretion and/or blood levels of transforming growth factor-β (TGF-β).

In embodiments of the invention relates to a TGF-β inhibitor for use in the treatment of virus-induced hyperinflammation, wherein the patient is preferably suffering from/experiencing elevated (above-normal/-healthy) expression, excretion and/or blood levels of transforming growth factor-β (TGF-beta).

In some specific embodiments, TGF-β and/or interferon may even be elevated only locally in individual organs or tissues, wherein viral reactivation (e.g., EBV or other HHV1-8 reactivation) may also only occur locally in individual organs or tissues.

In embodiments the virus-induced hyperinflammation is a severe acute hyperinflammatory shock or a multisystem inflammatory syndrome, or a (chronic) reactivation of Herpesviridae. In embodiments the virus-induced hyperinflammation is a severe acute hyperinflammatory shock or a multisystem inflammatory syndrome, or a (chronic) reactivation of Herpesviridae, preferably in Long-COVID-19.

In some embodiments the reactivation of a virus and/or virus-induced hyperinflammation in a patient is associated with, or induced by a COVID-19 infection. In embodiments the reactivation of viruses in a patient is considered a sequelae of COVID-19 and/or is associated with a prior COVID-19 infection, preferably a past or cured infection. In embodiments the sequelae of COVID-19 comprise the reactivation of viruses in the subject, preferably of Herpesviridae (HHV1-8).

In embodiments the reactivation of a virus in a patient is diagnosed or incurs at least 1 week, preferably after 4-8 weeks, after the COVID-19 infection/post-infection.

In some embodiments the reactivation of a virus in a patient is associated with, or induced by an immunosuppression and/or an impaired immune system in the patient. In embodiments the reactivation of viruses in a patient is diagnosed or incurs at least 1 day after, preferably after 1-100 days, of an immunosuppression and/or an impairment of the immune system in the patient. In embodiments the immunosuppression may be induced/caused by a pharmaceutical treatment (e.g., cancer therapy, immunosuppression/-modulation therapy) and/or a disease (e.g., leukaemia, lymphoma) in a patient.

In embodiments the virus is Epstein Barr virus (EBV). In embodiments the virus is Cytomegalovirus (CMV). In embodiments the virus is Herpes zoster.

In embodiments herpes viruses are human herpes viruses, such as CMV (=HHV5), EBV (=HHV4), HSV-1/2 (HHV1/2), herpes zoster virus (VZV=HHV3) and HHV6-8, or other human herpes viruses.

In another aspect the present invention relates to a TGF-β inhibitor for use in the treatment of the reactivation of a virus in a patient, wherein the patient is suffering from or experiencing elevated (above-normal) expression, excretion and/or blood levels of transforming growth factor-β (TGF-beta).

In certain embodiments of the present invention the patient is suffering from or experiencing elevated (above-normal) expression, excretion and/or blood levels (concentration in the blood) of transforming growth factor-β (TGF-β) and interferon.

In embodiments the reactivation of a virus and/or virus-induced hyperinflammation in a patient is associated with, or induced by a COVID-19 infection. Preferably a previous or former COVID-19 infection. In embodiments the virus is a human herpes virus (Herpesviridae).

In another aspect the present invention relates to a T-cell for the use in the treatment of virus reactivation (e.g., Herpesviridae or COVID-19) and/or SARS-CoV-2-induced long-term symptoms or sequelae, wherein the T-cells are/have been treated (e.g., by TGF-β inhibition) and/or engineered to be non-sensitive to TGF-β and/or immunosuppression, and preferably express CD4 and/or CD8. Preferably the T cell for the use in a treatment according to the invention is a population of T-cells.

In embodiments the T-cell for the use according to the invention has been stimulated according to a method described herein.

In embodiments the present invention relates to a T-cell for the use in the treatment of virus reactivation (e.g., Herpesviridae) in a patient, wherein the T-cells are/have been treated (e.g., by TGF-β inhibition) and/or engineered to be non-sensitive to TGF-β and/or immunosuppression, and preferably express CD4 and/or CD8.

In embodiments the present invention relates to a T cell for the use in the treatment of SARS-CoV-2-induced long-term symptoms or sequelae, wherein the T-cells are/have been treated (e.g., by TGF-β inhibition) and/or engineered to be non-sensitive to TGF-β and/or immunosuppression, and preferably express CD4 and/or CD8.

In some embodiments the T-cell used in said treatment is an autologous T-cell (a cell derived from the patient himself) of the treated patient.

In embodiments the genetic engineering may involve the genetic modification of a TGF- β receptor, e.g., involving its knock-out or the induction of (point) mutation(s) in the receptor, wherein the engineering preferably leads to the impairment and/or abduction of TGF-β (receptor) signalling. In embodiments a TGF-β receptor gene is genetically engineered using CRISPR-Cas. In embodiments the genetic engineering may involve the genetic modification of the TGF-β receptor 1 and/or 2 and/or 3.

Hence, in embodiments T-cells from a patient may be obtained and treated with/subjected to TGF-β (signalling) blockade or inhibition, and are subsequently or in parallel restimulated with the respective virus that is reactivated in a patient or fragments thereof. Subsequently, such stimulated T-cells may be administered to the same patient (autologous) or to a suitable patient suffering from viral reactivation and/or elevated levels of TGF-β. In embodiments the T-cells are genetically engineered to be non-sensitive to TGF-β and/or immunosuppression instead or in addition to TGF-β blockade/inhibition. In embodiments the engineering involves the modification of the TGF-β receptor, e.g., by the introduction of mutations or know-out (e.g., by deletion or mutation of the respective gene) of the receptor.

In embodiments viral reactivation in a patient, such as CMV (=HHV5), EBV (=HHV4), HSV-1/2 (HHV1/2), herpes zoster virus (VZV=HHV3) and HHV6-8 or other human herpes viruses, are associated with a previous COVID-19 infection. In embodiments the viral reactivation in a patient, such as CMV (=HHV5), EBV (=HHV4), HSV-1/2 (HHV1/2), herpes zoster virus (VZV=HHV3) and HHV6-8 or other human herpes viruses reactivation, occurs post-COVID-19 infection. In embodiments the viral reactivation in a patient, such as CMV (=HHV5), EBV (=HHV4), HSV-1/2 (HHV1/2), herpes zoster virus (VZV=HHV3) and HHV6-8 or other human herpes viruses reactivation, is classified as a post-COVID-19-sequelae.

In embodiments the systemic hyperinflammation in a patient is associated with increased levels of TGF-β expression/secretion/blood levels and/or viral reactivation in the patient.

In embodiments the TGF-β inhibitor is a chemical, pharmaceutical compound or small molecule compound that inhibits TGF-β signalling, expression, activation, secretion and/or decreases (reduces) TGF-β blood levels in a patient. In embodiments the reduction in active TGF-β blood levels in a patient may be accomplished by inhibition of the expression, secretion, the inactivation, inhibition, degradation, binding and/or removal of TGF-β from the blood stream of a patient.

In embodiments the TGF- β inhibitor is an inhibitor of the TGF-β (superfamily) signalling pathway. In embodiments the TGF- β inhibitor is a SMAD inhibitor and/or an inhibitor of MAPK (-signalling).

In embodiments, a TGF-β (pathway) inhibitor is an inhibitor of a transforming growth factor-β receptor (TGF-βR). The term TGFβ pathway inhibitor may be used interchangeably with an inhibitor of TGF-β signalling, an inhibitor of the TGF-β pathway. In embodiments, the TGF-β pathway inhibitor is an inhibitor of SMAD signalling.

In embodiments the inhibitor of transforming growth factor-β (signalling) is an inhibitor of transforming growth factor-β receptor (TGF-βR). In embodiments the inhibitor of transforming growth factor-β is a small molecule. In embodiments the inhibitor of TGF-β pathway is selected from SB431542, SB505124, SB525334, SD-208, A-83-01, R-268712, K02288, TGFβRI-IN-3, AUDA, Sulfasalazine, SB505124, BIBF-0775, LY2109761, GW788388, Dorsomorphin, Pirfenidone, RepSox, LY364947, TP0427736, LY2157299, LY2109761, LDN-193189, LDN-212854, LDN-214117, ML347, Vactosertib,, SIS3, PD, ITD-1, TA-02, DMH1, Halofuginone, Lycopus, Ginsenoside Rh4, GW788388.

In embodiments the TGF- β inhibitor is an antibody against TGF-β1 and/or TGF-β-2 and/or TGF-β-3. In embodiments the TGF-β inhibition (by the TGF- β inhibitor) may be a TGF-beta blockade, e.g., by an anti- TGF-β antibody.

In another aspect the present invention relates to a method for stimulating T cells comprising
a. Selecting a population of, preferably CD4/CD8 expressing, T cells from a sample, preferably a blood sample from a patient (autologous T cells),
b. Optionally genetically engineering said T cells to be resistant or insensitive to TGF-β stimulation (e.g., by modifying or deleting the TGF-β receptor),
c. Providing a population of antigen-presenting cells, such as e.g., B cells, optionally wherein the antigen-presenting cells are antigen-presenting cells from a blood sample of a patient,
d. Optionally incubating the population of antigen-presenting cells, e.g., B cells, with viral particles or parts thereof (e.g., Epstein-Barr Virus (EBV) or another herpes virus (HHV1-8)), or modifying the antigen-presenting cells to present viral antigens,
e. Stimulating the population of T cells,
   wherein the stimulation preferably comprises co-culture of the population of T cells with the population of antigen-presenting cells, e.g., B cells, provided in step c.,
   wherein during step a. and/or e. the T cells are incubated with a TGF- β inhibitor, and/or
   wherein the T-cells have been genetically engineered to be TGF-β insensitive.

In embodiments the method for stimulating T cells comprises the steps of
a. Providing a population of, preferably CD4/CD8 expressing, T cells,
b. Optionally incubating the T cells with a TGF- β inhibitor and/or genetically engineering the T cells to be resistant or insensitive to TGF-β stimulation (e.g., by modifying or deleting the TGF-β receptor),
c. Providing a population of antigen-presenting cells, preferably comprising B cells,
d. Incubating the population of antigen-presenting cells, preferably comprising B cells, with viral particles or parts thereof (e.g., EBV or another herpes virus (HHV1-8)),
e. Stimulating the population of T cells, comprising co-culturing the population of T cells with the population of antigen-presenting cells provided in step c.

In embodiments the method for stimulating T cells comprises the steps of
a. Providing a population of, preferably CD4/CD8 expressing, T cells,
b. Optionally genetically engineering said T cells to be resistant or insensitive to TGF-β stimulation (e.g., by modifying or deleting the TGF-β receptor),
c. Providing a population of antigen-presenting cells, such as e.g., B cells, optionally wherein the antigen-presenting cells are antigen-presenting cells from a blood sample of a patient,
d. Optionally contacting the population of antigen-presenting cells, e.g., B cells, with viral particles or parts thereof (e.g., EBV or another herpes virus (HHV1-8)), or modifying the antigen-presenting cells to present viral antigens,
e. Stimulating the population of T cells,
   wherein the stimulation preferably comprises co-culturing or contacting the population of T cells with the population of antigen-presenting cells, e.g., B cells, provided in step b.,
   wherein during step a. and/or e. the T cells are brought into contact with a TGF- β inhibitor, and/or
   wherein the T-cells have been genetically engineered to be TGF-β insensitive.

In another embodiments the method for stimulating T cells comprises
a. Providing a cell population comprising T cells,
b. Contacting said cell population with one or more antigens and/or antigen-presenting cells *in vitro,*
   wherein during step a. and/or b. the T cells are incubated with a TGF- β inhibitor, and/or
   wherein the T-cells have been genetically engineered to be TGF-β insensitive.

In embodiments the method for stimulating T cells according to the invention are employed for stimulating a T-cell for the use according to the invention (before its use according to the invention). Hence, in one aspect the present invention relates to a population of T cells produced (stimulated) by the method according to the invention.

In embodiments of the afore methods for stimulating T cells the viral particles or parts thereof are herpes virus particles or parts thereof, such as EBV or another herpes virus (HHV1-8). In embodiments the viral antigens are herpes virus antigens, such as antigens of EBV or another herpes virus (HHV1-8).

In embodiments of the afore methods for stimulating T cells the viral particles or parts thereof are COVID-19 virus particles or parts thereof. In embodiments the viral antigens are COVID-19 virus antigens (antigens of COVID-19 virus).

This aspect of the invention is at least partially based on the surprising finding of the inventors, that hyperinflammation in patients (e.g., MIS or MIS-C induced by previous viral infections) affects memory T-cell function. The inventors surprisingly found, that during acute MIS-C, both CD4+ and CD8+ T-cells co-cultured with antigen presenting cells and various viral peptides showed functional impairment by reduced expression of the early activation marker CD69 (see e.g., Fig. 2d). Even specific reactivation by these viral peptides was impaired during the acute phase of MIS-C (see Examples). Given the strong TGF-β instruction (stimulation) of T-cells in MIS-C (e.g., see Fig. 2a) and because TGF-β has been implicated in terminating immune responses before, the inventors hypothesized that the observed impairment in memory T-cell reactivation could be reproduced with T-cells derived from healthy donors when stimulated in the presence of TGF-β. Indeed, the inventors reviled that the addition of TGF-β significantly impaired T-cell reactivity (e.g., see Fig. 9b-c). In summary the inventors showed that observed impairment of memory T-cell reactivation could be effectively reversed by blocking TGF-β. Therefore, in embodiments, besides the inhibition of TGFbeta in a patient, a virus-induced hyperinflammation or (post-) COVID-19 symptoms or sequelae in a patient suffering from or experiencing elevated (above-normal) expression, excretion and/or blood levels of transforming growth factor-β (TGF-β) and/or interferon, may be treated by administrating either T cells derived from a healthy donor or derived from the patient itself (autologous T cells) that have preferably been stimulated according to the methods of the present invention and/or treated by TGF-β inhibition according to the methods of the present invention. In embodiments the administration of T cells treated/stimulated according to the invention is considered to at least partially restore the CD4+ and/or CD8+ T-cell activity in said patient, thereby reducing/treating the virus-induced hyperinflammation or (post-) COVID-19 symptoms or sequelae in said patient.

To improve in embodiments the resistance of the administered T cells to the elevated (above-normal) expression, excretion and/or blood levels of transforming growth factor-β (TGF-β) and/or interferon in a patient, the T cells may be treated with a TGF-β inhibitor and/or be genetically engineered, e.g., by a knock-down/knock-out of one or more TGF-β receptors or receptors in TGF-β signalling, to be resistant or insensitive to TGF-β stimulation. This is especially advantageous, as the inventors revealed (see Examples herein) that TGF-β significantly impaired T-cell reactivity and memory T-cell reactivation in a patient, but also that the observed impairment of memory T-cell reactivation could be effectively reversed by blocking TGF-β.

In embodiments methods and means for engineering or stimulating T-cells may be performed as described before in Russo et al., 2022 (Wendering et al., Tacrolimus-resistant SARS-CoV-2-specific T cell products to prevent and treat severe COVID-19 in immunosuppressed patients. Mol Ther Methods Clin Dev. 2022 Jun 9;25:52-73. doi: 10.1016/j.omtm.2022.02.012. Epub 2022 Feb 26. PMID: 35252469; PMCID: PMC8882037*.)* which is incorporated by reference herein.

Contacting a cell population of T cells or comprising the same to an antigen may occur by any suitable means in the art. For example, a cell population obtained from a subject may be collected, optionally cultivated, and maintained *in vitro* or *ex vivo,* preferably in a liquid medium, into which an antigen can be applied. The antigen may be immobilized to a solid phase, which is either brought into contact with the relevant T cell containing population or with a population of antigen-presenting cells, e.g., B cells. Means for obtaining samples comprising T cells and/or antigen presenting cells, and means for collecting and/or cultivating T cells and/or antigen presenting cells, are known in the art.

The invention relates in another aspect to a method of treating virus-induced hyperinflammation or (post-) COVID-19 symptoms or sequelae in a subject, wherein the subject is suffering from or experiencing elevated (above-normal) expression, excretion and/or blood levels of transforming growth factor-β (TGF-β), comprising administering a therapeutical effective amount of a TGF-β inhibitor to the subject in need thereof.

Each optional or preferred feature of the invention that is disclosed or described in the context of one aspect of the invention is herewith also disclosed in the context of the other aspects of the invention described herein.

All features disclosed in the context of the TGF-β inhibitor for use in the treatment of virus-induced hyperinflammation or (post-) COVID-19 symptoms or sequelae, wherein the patient is suffering from or experiencing elevated (above-normal) expression, excretion and/or blood levels of transforming growth factor-β (TGF-β) also relate to and are herewith disclosed also in the context of the TGF-β inhibitor for use in the treatment of virus-reactivation in a subject, the in vitro method for stimulating T cells of the invention, the methods of treatment of the invention and the T cells for use in the treatment of virus-induced hyperinflammation or (post-) COVID-19 symptoms or sequelae in a subject, and vice versa.

### DETAILED DESCRIPTION OF THE INVENTION

All cited documents of the patent and non-patent literature are hereby incorporated by reference in their entirety.

The present invention is directed in one aspect to the targeted intervention involving TGF-β blockade for the management of MIS, particularly MIS-C, and also other inflammatory (post-) COVID-19 sequelae. In embodiments beyond (post-)COVID-19 sequelae, therapies comprising the administration of autologous EBV or SARS-CoV-2-specific T-cells engineered to be non-sensitive to TGF-β and/or immunosuppression (e.g., as described in Peter, Wendering et al., 2022) can alleviate undesirable virus-induced hyperinflammation.

As used herein, the "patient" or "subject" may be a vertebrate. In the context of the present invention, the term "subject" includes both humans and animals, particularly mammals, and other organisms. Herein a "patient" or "subject" is preferably a human. In embodiments the human patient or subject is a child. In embodiments the human patient or subject is an adult. Herein, the terms "patient" and "subject" may be used interchangeably.

In the context of the present invention, the term "child" may refer the following age groups: Newborns up to the completed 28th day of life, infants from the beginning of the 29th day of life up to the completed 12th month of life, toddlers from the beginning of the 2nd to the completed 3rd year of life and/or children from the beginning of the 4th to the completed 18th year of life. In general, in the context of the present invention, the term "child" may be used as a generic term for all persons under 21 years of age, preferably under 16 years, more preferably under 12 years of age. On embodiments the subject is older than 16, or even older than 21 years. In the context of the present invention virus-induced hyperinflammation or (post-)COVID-19 symptoms or sequelae may occur in a subject independent of its age or at any age.

Transforming growth factor beta (TGF-β) is a cytokine belonging to the transforming growth factor superfamily comprising TGF-β 1 to 3 and numerous other signalling proteins. TGFβ proteins are produced by all white blood cell lines. Activated TGF-β forms a serine/threonine kinase complex with other factors that binds to TGF-β receptors. The transforming growth factor beta (TGFB) signalling pathway is involved in cellular processes, such as cell differentiation, cell growth, cell migration, apoptosis, cellular homeostasis, etc. TGFβ-family ligands bind to a 'type II receptor', thereby inducing the recruitment and phosphorylating of a 'type I receptor', which then phosphorylate receptor-regulated SMADs (R-SMADs). Activated SMADs subsequently bind the coSMAD SMAD4 and accumulate in the nucleus, to regulate the expression of target genes. SMADs (or Smads) in general are a family of structurally similar proteins that are the major signal transducers for transforming growth factor beta (TGFb; TGFbeta) superfamily receptors, which are critical for regulating cell development and growth.

Interferons (IFNs) are endogenous proteins or cytokines produced by a variety of cells in the inflammatory response to infection. Interferons play an important role in the innate immune response to viral infections. Elevated interferon may be a characteristic and/or indicative of prolonged and/or exuberant inflammatory response in a subject.

Herein, "elevated (above-normal/-healthy) expression, excretion and/or blood levels" of a protein or factor refers to an increased abundance, level or concentration of said protein or factor in a respective medium (e.g., body fluid, blood or plasma) that can - when compared to the abundance, level or concentration of said protein or factor in a healthy/normal individual - be regarded as higher or significant higher, thereby potentially indicating an abundance, level or concentration that is above-normal/above-healthy or indicative of a disease, abnormal or medical condition. Therefore, in embodiments an elevated (above-normal/-healthy) expression, excretion and/or blood levels of a protein or factor may be indicative of a disease, abnormal or medical condition. In some embodiments herein elevated (above-normal/-healthy) - compared to a healthy or normal (control) individual - expression, excretion and/or blood levels of transforming growth factor-β (TGF-β) (and optionally also of interferon) are indicative of a virus, herpes virus or COVID-19-induced hyperinflammation or (post-)COVID-19 symptoms or sequelae, in embodiments of a severe acute hyperinflammatory shock or a multisystem inflammatory syndrome, in the individual.

In the present invention "treatment" or "therapy" in general aims to obtain a desired physiological effect and/or pharmacological effect. The effect may be prophylactic in view of entirely or partially preventing a disease and/or a symptom, i.e., by reducing the risk of a subject having a disease or symptom or may be therapeutic in view of partially or entirely curing a disease and/or adverse effect of the disease.

In the present invention a "treatment" includes arbitrary treatments of diseases or conditions in mammals, in particular, humans, for example, the following treatments: a. prevention of onset of a disease, condition or symptom in a subject, b. inhibition of a symptom of a condition, that is, prevention of progression of the symptom, c. amelioration of a symptom of a condition, that is, induction of regression of the disease or symptom. In accordance with the various treatment methods of the present invention, a compound, composition or population of cells may be delivered to a subject in a manner consistent with conventional methodologies associated with management of the disorder for which treatment or prevention is sought. In accordance with the disclosure herein, a prophylactically or therapeutically effective amount of the compound and/or other biologically active agent is administered to a subject in need of such treatment for a time and under conditions sufficient to prevent, inhibit, and/or ameliorate a selected disease or condition or one or more symptom(s) thereof.

Dosage regimens can be adjusted to provide an optimum prophylactic or therapeutic response. A therapeutically effective amount is also one in which any toxic or detrimental side effects of the compound and/or other biologically active agent is outweighed in clinical terms by therapeutically beneficial effects. As used herein, a "therapeutically effective amount" of a compound or pharmaceutical composition includes, without limitation, the following amounts and ranges of amounts of about 0.001 mg/kg body weight to 50 mg/kg body weight, 0.01 mg/kg body weight to about 20 mg/kg body weight, such as about 0.05 mg kg to about 5 mg/kg body weight, or about 0.2 mg/kg to about 2 mg/kg body weight.

For a composition comprising T-cells according to the invention or embodiments thereof: (i) from about 1 × 10² to about 1 × 10⁸ cells/kg body weight; (ii) from about 1 × 10³ to about 1 × 10⁷ cells/kg body weight; (iii) from about 1 × 10⁴ to about 1 × 10⁶ cells/kg body weight; (iv) from about 1 × 10⁴ to about 1 × 10⁵ cells/kg body weight; (v) from about 1 × 10⁵ to about 1 × 10⁶ cells/kg body weight; (vi) from about 5 × 10⁴ to about 0.5 × 10⁵ cells/kg body weight; (vii) about 1 × 10³ cells/kg body weight; (viii) about 1 × 10⁴ cells/kg body weight; (ix) about 5 × 10⁴ cells/kg body weight; (x) about 1 × 10⁵ cells/kg body weight; (xi) about 5 × 10⁵ cells/kg body weight; (xii) about 1 × 10⁶ cells/kg body weight; and (xiii) about 1 × 10⁷ cells/kg body weight.

Dosages of the therapy according to the invention will depend on factors such as the condition being treated, the selected gene, the age, weight and health of the patient, and may thus vary among patients.

Compound, compositions or cells for administration to a subject can include at least one further pharmaceutically acceptable additive such as carriers, thickeners, diluents, buffers, preservatives, surface active agents and the like in addition to the molecule of choice. Pharmaceutical compositions can also include one or more additional active ingredients such as anti-inflammatory agents, anaesthetics, antimicrobial agents, and the like. The pharmaceutically acceptable carriers useful for these formulations are conventional. Remington's Pharmaceutical Sciences, by E. W. Martin, Mack Publishing Co., Easton, PA, 19th Edition (1995), describes compositions and formulations suitable for pharmaceutical delivery of the compounds herein disclosed.

Commonly, the nature of the carrier will depend on the respective mode of administration being used. For example, parenteral formulations usually contain injectable fluids that include pharmaceutically and physiologically acceptable fluids such as water, balanced salt solutions, physiological saline, aqueous dextrose, glycerol or the like as a vehicle. For solid compositions (for example, pill, tablet, powder, or capsule forms), conventional non-toxic solid carriers can include, for example, pharmaceutical grades of starch, mannitol, lactose, or magnesium stearate. In addition to biologically-neutral carriers, pharmaceutical compositions to be administered can contain minor amounts of non-toxic auxiliary substances, such as wetting or emulsifying agents, preservatives, and pH buffering agents and the like.

The compositions may be administered by routes including intravenous, intra-arterial, intramuscular, intraocular, subcutaneous, intra-articular, intraperitoneal, intrathecal, intracerebroventricular, or parenteral routes.

The compositions of the disclosure can alternatively contain as pharmaceutically acceptable carrier substances as required to approximate physiological conditions, such as pH adjusting and buffering agents, tonicity adjusting agents, wetting agents and the like, for example, sodium chloride, sodium acetate, sodium lactate, calcium chloride, potassium chloride, sorbitan monolaurate, and triethanolamine oleate. For solid compositions, conventional nontoxic pharmaceutically acceptable vehicles can be used which include, for example, lactose, pharmaceutical grades of mannitol, magnesium stearate, starch, sodium saccharin, talcum, cellulose, glucose, magnesium carbonate, sucrose, and the like.

Coronaviruses are a group of related viruses that cause diseases in mammals and birds. The scientific name for coronavirus is *Orthocoronavirinae* or *Coronavirinae.* Coronavirus belongs to the family of *Coronaviridae.* The family is divided into *Coronavirinae* and *Torovirinae* sub-families, which are further divided into six genera: *Alphacoronavirus, Betacoronavirus, Gammacoronavirus, Deltacoronavirus, Torovirus,* and *Bafinivirus.* While viruses in the genera *Alpha corona viruses* and *Betacoronaviruses* infect mostly mammals, the *Gammacoronavirus* infect avian species and members of the *Deltacoronavirus* genus have been found in both mammalian and avian hosts.

In humans, coronaviruses cause respiratory tract infections that can be mild, such as some cases of the common cold, and others that can be lethal, such as SARS, MERS, and COVID-19. Various species of human coronaviruses are known, e.g., without limitation, Severe acute respiratory syndrome coronavirus (SARS-CoV), Severe acute respiratory syndrome coronavirus 2 (SARS-CoV-2), Middle East respiratory syndrome-related coronavirus (MERS-CoV), Human coronavirus OC43 (HCoV-OC43), of the genus β-CoV, Human coronavirus HKU1 (HCoV-HKU1), of the genus β-CoV, Human coronavirus 229E (HCoV-229E), α-CoV, Human coronavirus NL63 (HCoV-NL63), α-CoV. Coronaviruses vary significantly in risk factor. Some are relatively harmless, such as the common cold. Coronaviruses cause colds with major symptoms, such as fever, and a sore throat, e.g., from swollen adenoids, occurring primarily in the winter and early spring seasons. Coronaviruses can cause pneumonia (either direct viral pneumonia or secondary bacterial pneumonia), bronchitis (either direct viral bronchitis or secondary bacterial bronchitis) and also Severe acute respiratory syndrome (SARS). A COVID-19 infection can progress through several disease phases. In a late phase of the disease a hyperinflammatory phase of disease may occur in some patients (phase III), wherein immunological processes are the primary cause, while viral replication is undetectable. Presently, the only and mostly unspecific treatment of this condition are anti-inflammatory drugs such as systemic glucocorticoids. Indicators of hyperinflammation comprise, without being limited to fever, elevated levels of ferritin, cytokines, and/or CRP concentrations, excessive release of proinflammatory cytokines, rapid respiratory deterioration, changes in blood levels of certain immune cells, and multiple organ dysfunction.

Post sequelae of SARS-CoV-2 or "COVID-19 sequelae", also known as post-Covid syndrome or long COVID (long-COVID-19), have been defined as a diverse constellation of signs and symptoms affecting multiple organs that continue to occur weeks or months after the initial COVID-19 infection. Although respiratory tract infection, muscle and joint aches and/or fever are considered some of the primary symptoms of an acute/ongoing COVID-19 infection. COVID-19 is considered a systemic multi-organ condition involving the lungs, vasculature, heart, and even the brain and other organ systems. The assignment of symptoms to long COVID and (post) COVID19 sequelae is currently still ongoing, as the field of research on (post/long) COVID sequalae and symptoms is still evolving, and new clinical findings and studies are constantly reported. At present, post COVID19 sequelae may comprise, without being limited to, (systemic and/or chronic) hyperinflammation in multiple tissues and/or organs, fever, fatigue, muscle and/or joint pain, impaired ability to (physically) exercise, neuromuscular syndromes, cognitive impairments, neuropsychiatric disorders, gastrointestinal dysregulation, vascular endothelial dysregulation, multisystemic tissue damage including cardiopulmonary syndromes, lung damage, acute encephalitis, fibrosis, myocardial infarction, hepatobiliary damage renal damage and/or failure and stroke. Post COVID-19 sequelae may comprise persistent symptoms beyond one month after acute infection, which cannot be explained by another diagnosis, and occur after an acute COVID-19 infection. COVID-19-induced (systemic) hyperinflammation is considered a (post-) COVID-19 symptom or sequelae. In embodiments the COVID-19-induced hyperinflammation is a severe acute hyperinflammatory shock or a multisystem inflammatory syndrome. (Dormant) virus reactivation, e.g., herpes virus reactivation, may in embodiments also be considered a (post-) COVID-19 symptom or sequelae. In some embodiments the virus- and/or COVID-19-induced hyperinflammation is associated with, or accompanied by (dormant) virus reactivation, e.g., herpes virus reactivation.

Therefore, in embodiments long-COVID-19 or (post-)COVID-19 symptoms or sequelae comprise one or more of, without being limited to, (systemic and/or chronic) hyperinflammation in multiple tissues and/or organs, severe acute hyperinflammatory shock, multisystem inflammatory syndrome, multisystem inflammatory syndrome in children (MIS-C), fever, fatigue, (dormant) virus reactivation, e.g., herpes virus reactivation, muscle and/or joint pain, impaired ability to (physically) exercise, neuromuscular syndromes, cognitive impairments, neuropsychiatric disorders, gastrointestinal dysregulation, vascular endothelial dysregulation, multisystemic tissue damage including cardiopulmonary syndromes, lung damage, acute encephalitis, fibrosis, myocardial infarction, hepatobiliary damage, renal damage and/or failure and stroke.

Multisystem inflammatory syndrome in children (MIS-C) is a rare condition associated with SARS-CoV-2 infection and commonly occurs 2-6 weeks after an infection with SARS-CoV-2. MIS-C may occur, even after a very mild or symptom-less SARS-CoV-2 infection. MIS-C causes various internal and external body parts to become inflamed, including the lungs, kidneys, heart, brain, skin, eyes, and/or the gastrointestinal tract. MIS-C may be a serious, even fatal condition, but most diagnosed children recover after treatment. Symptoms are currently considered to comprise stomach pain, bloodshot eyes, vomiting, diarrhoea, skin rash, low blood pressure, dizziness and/or or light-headedness.

T cells are a type of lymphocyte, namely a type of leukocytes (white blood cells) and play an important role in the immune system and in adaptive immune response. T cells may be distinguished from other lymphocytes by the presence of a T cell receptor (TCR) on their cell surface. Groups of specific, differentiated T cell subtypes have diverse functions in controlling and modifying the immune response. One of said functions is immune-mediated cell death, and it is carried out by two major subtypes: CD8+ "killer" and CD4+ "helper" T cells. One population of T cells, the CD4+ T cells, function as "helper cells". CD4+ helper T (TH) cells function by further activating memory B cells and cytotoxic T cells, which leads to a larger immune response. CD8+ T cells, also known as "killer T cells", are cytotoxic, meaning that they are able to kill e.g. virus-infected cells, as well as cancer cells. CD8+ T cells are also able to use small signalling proteins, known as cytokines, to recruit other types of cells when mounting an immune response. Regulatory T cells are another distinct population of T cells that provide the critical mechanism of tolerance, whereby immune cells are able to distinguish invading cells from "self". This prevents immune cells from inappropriately reacting against one's own cells, known as an "autoimmune" response. For this reason, these regulatory T cells have also been called "suppressor" T cells. These same regulatory T cells can also be co-opted by cancer cells to prevent the recognition of, and an immune response against, tumour cells. The present invention may comprise any given population of T cells, as described herein, and as known to a skilled person.

Generally, the innate immune system is the first line of defense against pathogens, which is a non-specific immune response aiming to immediately prevent the spread of foreign pathogens throughout the body. The innate immune response consists of chemical, physical and cellular defenses against pathogens. On the other hand, the acquired immune system is the second line of defense against pathogens. Acquired immunity is also referred to as adaptive immunity or specific immunity and is specific to the respective pathogen infecting a subject.

As used herein, "diagnosis" in the context of the present invention relates to the recognition and (early) detection of a clinical condition of a subject associated with a SARS-CoV infection. Also the assessment of the possibilities of developing adverse symptoms may be encompassed by the term "diagnosis".

"Prognosis" relates to the prediction of an outcome or a specific risk for a subject based on a clinical condition of a subject linked to a previous viral, e.g., a herpes virus or SARS-CoV infection. This may also include an estimation of the chance of recovery or the chance of an adverse outcome for said subject.

As used herein, the terms "comprising" and "including" or grammatical variants thereof are to be taken as specifying the stated features, integers, steps or components but do not preclude the addition of one or more additional features, integers, steps, components or groups thereof. This term encompasses the terms "consisting of" and "consisting essentially of. Thus, the terms "comprising"/"including"/"having" mean that any further component (or likewise features, integers, steps and the like) can/may be present. The term "consisting of means that no further component (or likewise features, integers, steps and the like) is present.

The instant disclosure also includes kits, packages and multi-container units containing the herein described compositions, active ingredients, and/or means for administering the same for use in the prevention and treatment of diseases and other conditions in mammalian subjects.

### FIGURES

The invention is further described by the following figures. These are not intended to limit the scope of the invention, but represent preferred embodiments of aspects of the invention provided for greater illustration of the invention described herein.

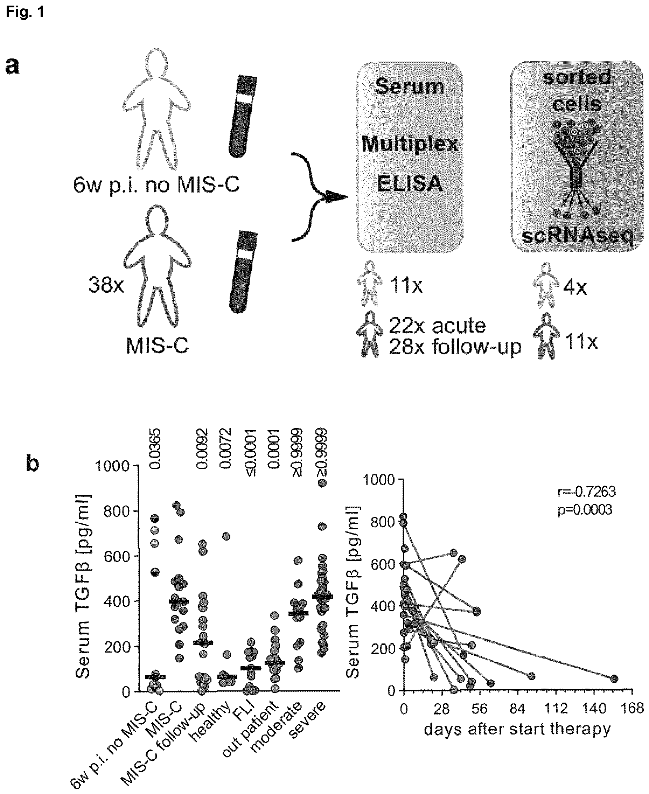

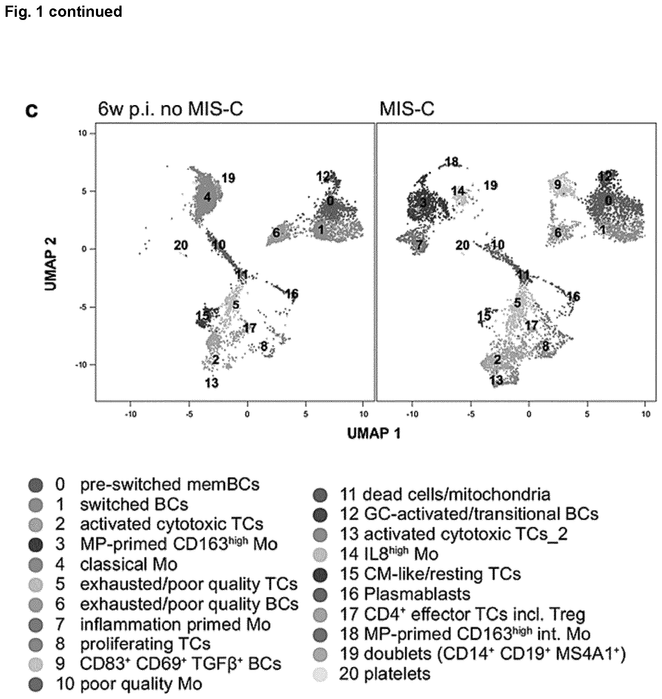

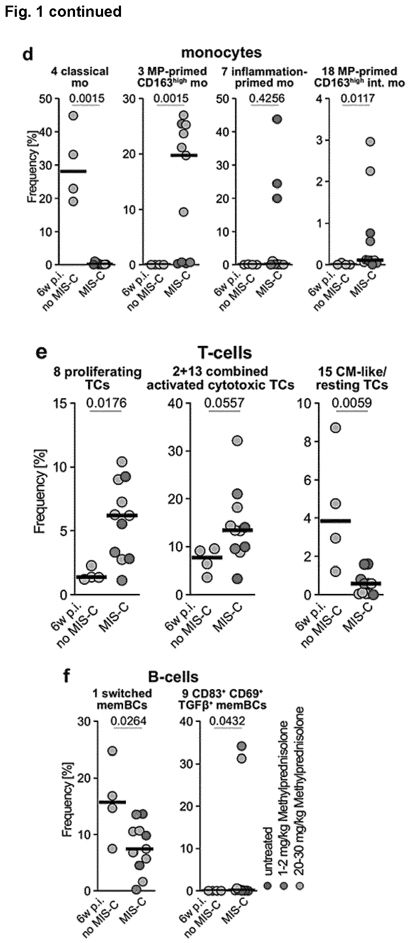

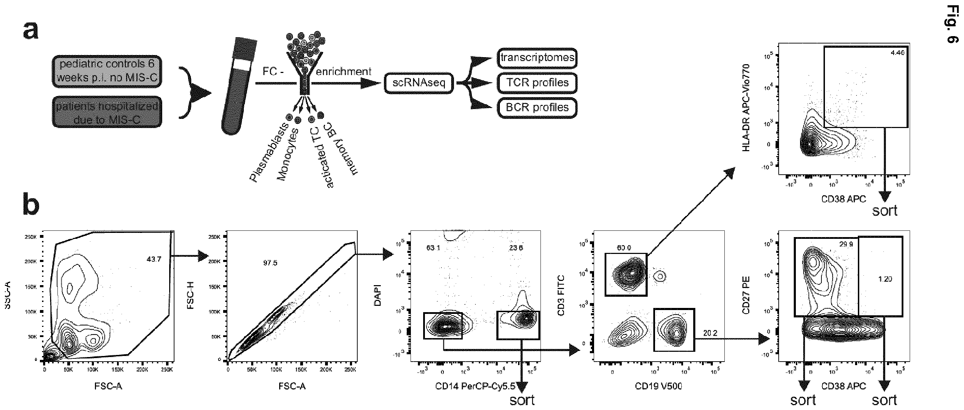

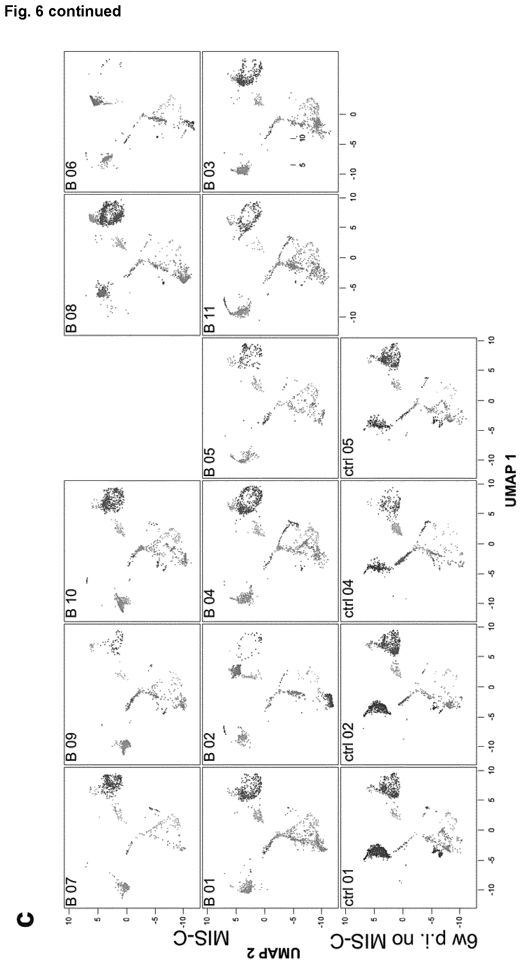

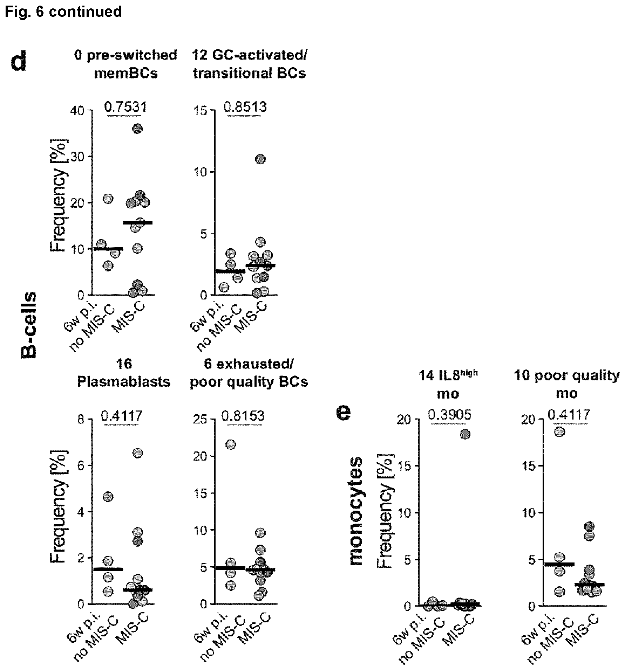

**Figure 1****: Cytokine profile and impact on immune cell compartments in MIS-C.** a Schematic illustration of patients included in cytokine profiling and single-cell RNA-sequencing (scRNAseq). **b** Serum TGF-β levels from 22 patients with MIS-C during the first 7 days of hospitalisation (n=22) and during follow-up (n=28 time points, 23 patients) set in relation to paediatric controls 6 weeks post-infection with SARS-CoV-2 (6w p.i. no MIS-C, n=6 healthy and 5 with underlying rheumatic condition), and follow ups of MIS-C patients 7 healthy people aged less than 30 years, 13 patients with flu-like illnesses (FLI) (n=13) and patients with COVID-19 during the first two weeks of infection (out patient n=19; moderate n=12; severe n=34) we previously published(Witkowski, Tizian et al. 2021). Additionally on the right plot, serum levels overtime (0 indicates day of hospitalisation and initiation of treatment) in individuals with MIS-C are indicated and decline in TGF-β serum levels was correlated to time after initiation of treatment. **c-f** scRNA-seq of PBMC enriched by FACS for monocytes, HLA-DR^{high}, CD38⁺ T-cells and CD27⁺ B-cells of n=4 paediatric controls 6 weeks post infection with SARS-CoV-2 and n=11 patients hospitalised for MIS-C. The sorting strategy is depicted in Fig. 6b. **c** UMAP of 6589 cells from p.i. no MIS-C (n=4 individuals) and 34794 cells from MIS-C (n=11 individuals) separated by cohort and rarefied to depict equal numbers for both groups. Colours indicate unsupervised clustering. Individual patients' UMAP are depicted in Fig. 6c. **d-f** Frequencies of cells per cluster in both groups. Treatment with methylprednisolone is colour-coded. Black horizontal lines depict the median. Frequencies of remaining clusters are depicted in Fig. 6d. In **b,** a non-parametric ANOVA (Kruskal-Wallis test) was used followed by a Dunn's multiple comparison test with correction for multiple comparisons, comparing each group to MIS-C patients and for time-correlation, a spearman-correlation was used. In **d-f,** p values were calculated by two-tailed Mann-Whitney U-tests.

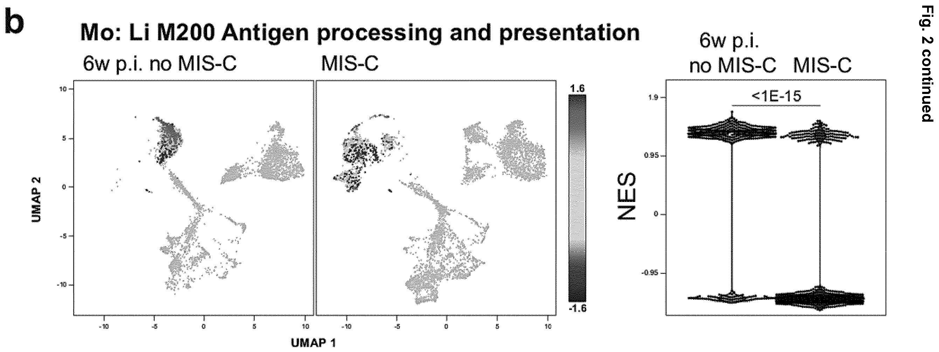

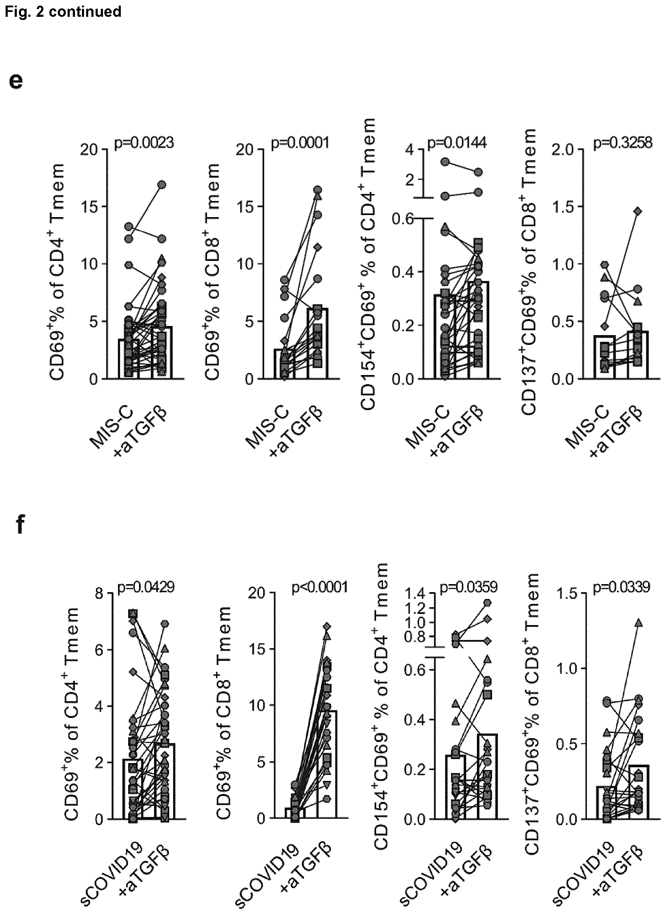

**Figure 2****: impaired T-cell reactivity during acute phase of MIS-C is induced by TGF-β. a** GSEA using a previously defined TGF-β gene set(Witkowski, Tizian et al. 2021) applied to T-cells and **b** for the gene set "Li M200 antigen processing and presentation"(Li, Rouphael et al. 2014) applied to monocyte clusters depicted as both a UMAP and a dot plot. **c** Schematic overview of T-cell reactivation assays. **d** Frequencies of overall activation (CD69⁺) and antigen-specific reactivation (CD69⁺ and CD154⁺ or CD137⁺) of CD4⁺ and CD8⁺ T-cells from patients with MIS-C during the acute phase and at follow-up after symptoms resolved (n=8 patients and n=5 different viral peptides). **d-f** Frequencies of overall activation and antigen-specific reactivation of CD4⁺ and CD8⁺ T-cells from healthy donors (n=6) treated with serum from c MIS-C patients (n=7) or **c** severe COVID-19 patients (n=5) with or without TGF-β1/2/3 neutralising antibody. p-values were calculated using unpaired **(a+b)** or paired **(d-f)** two-tailed Mann-Whitney U-tests.

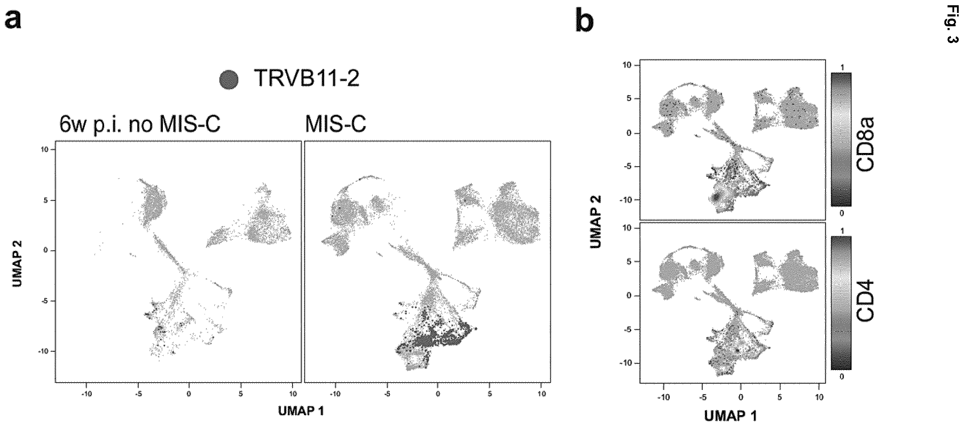

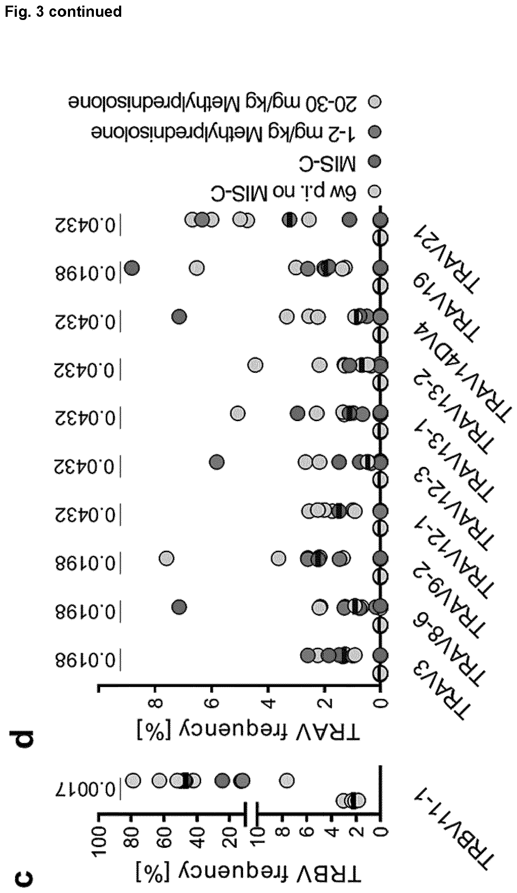

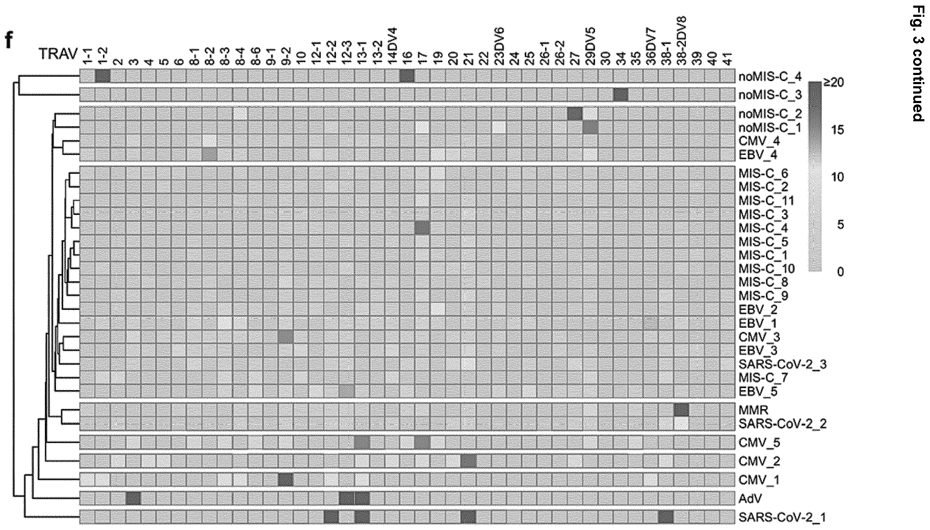

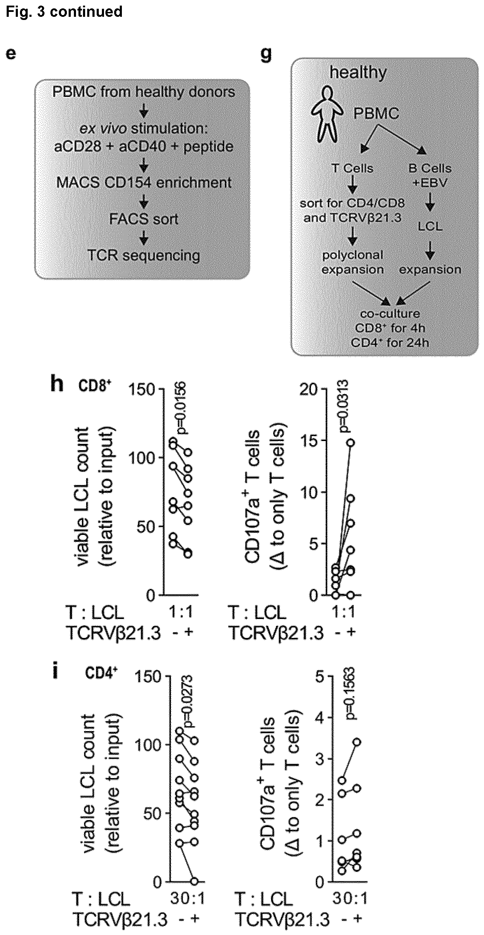

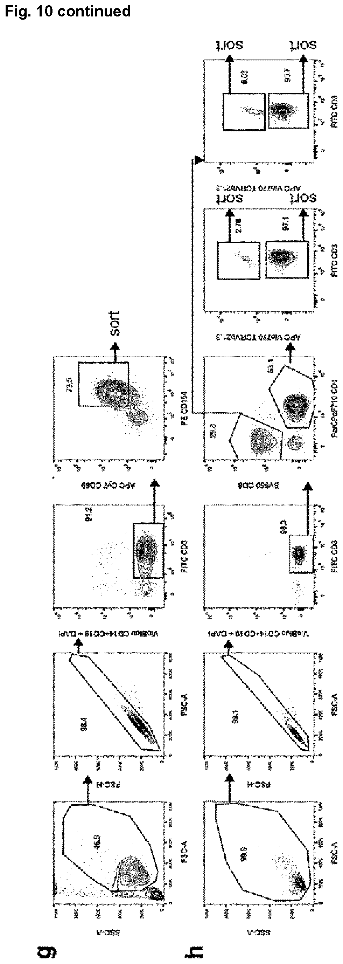

**Figure 3****: TCR repertoires of oligoclonal expanded T-cells in MIS-C show overlap with EBV-specific TCR repertoires, a** *TRBV11-2* positive T-cells are highlighted (in red) in the UMAP enriched activated cells of paediatric controls 6 weeks p.i. and MIS-C patients (see Figure 1c). **b** UMAP depicting expression of CD8a and CD4 genes (CD4 expression only on T-cells is depicted for better overview). **c** Frequency of *TRBV11-2* positive T-cells among total T-cells for paediatric controls 6 weeks p.i. (lowest *TRAV* frequency) and MIS-C patients during the acute phase and **d** Frequencies of indicated *TRAVs* associated to *TRBV11-2* (n=4 controls and n=11 MIS-C patients). Treatment with methylprednisolone is colour (greyscale)-coded, MIS-C indicated sampling before start of treatment. The treatment with 20-30 mg/kg Methylprednisolone resulted on average the highest *TRAV* frequency. **e** Experimental set up for single-cell TCR sequencing and subsequent unsupervised clustering of TCR repertoires, **f** Using the ARTE (Bacher, Schink et al. 2013) assay (Figure 10g) TCR-repertoires of T-cells specific for EBV (n=5), CMV (n=5), SARS-CoV-2 (n=3), MMR (n=1) and AdV (n=1) from healthy donors were sequenced. Frequencies of *TRAVs* associated to TRBV11-2-positive T-cells were determined and depicted in the heat map. Unsupervised clustering was performed using the R package pheatmap. **g** Experimental set up of T-cell killing assay, **h-i** PBMC from healthy donors (n=4 individuals at each 2 different time points) were MACS and FACS enriched for CD4⁺ and CD8⁺ T-cells, with or without TCRVβ21.3 expression, and polyclonally expanded using CD3/CD28 coated beads. PBMC from the same donors were transfected with EBV to generate a lymphoblastic cell line (LCL). T-cells and LCL from the same donor were co-cultured in the following conditions: **h** CD8⁺ T-cells at a ratio of 1:1 for 4 hours; **i** CD4⁺ T-cells at a ratio of 30:1 for 24 hours. After incubation, viable LCL were counted by flow cytometry and normalised to LCL incubated without T-cells. Additionally, CD107a expression was determined by flow cytometry and normalised to T-cells incubated without LCL. p values for **c-d** were determined by two-sided Mann-Whitney U-tests and for **h-i,** paired two-tailed Mann-Whitney U-tests were used.

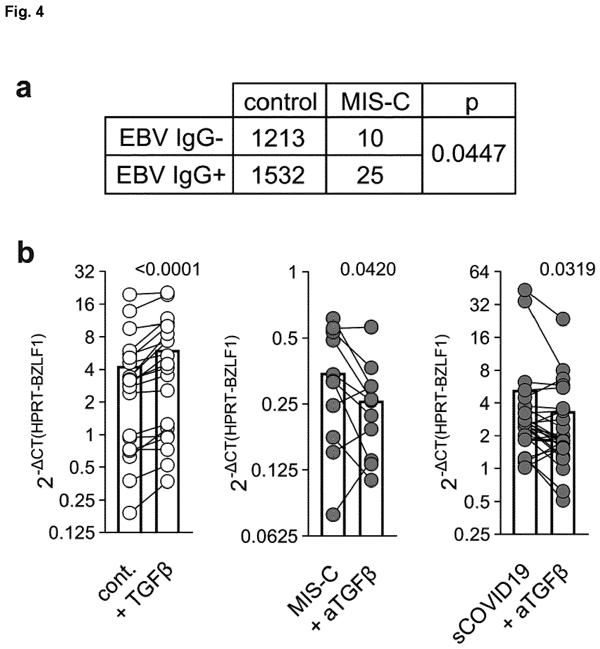

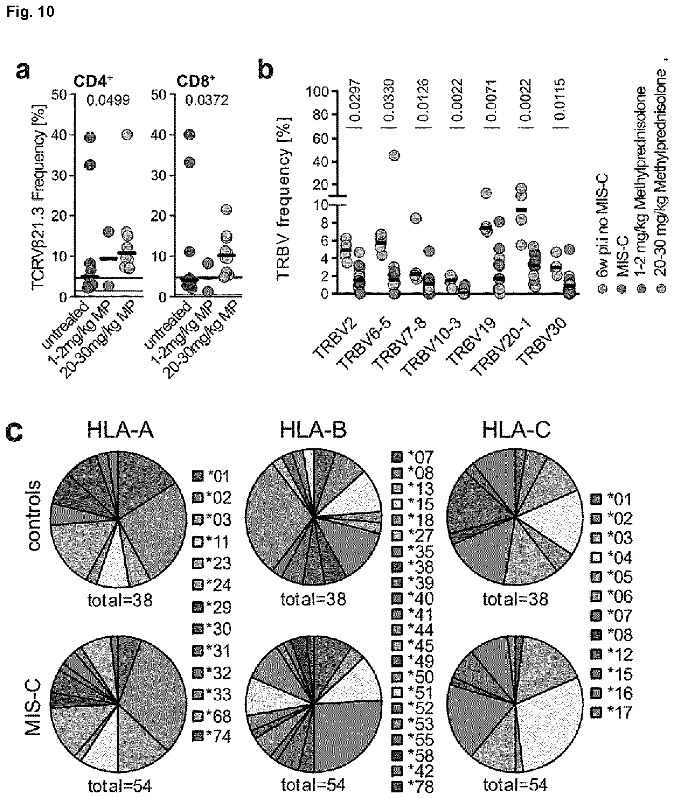

**Figure 4****: EBV reactivation is prominently observed in MIS-C.** a Number of EBV-specific IgG seropositive and seronegative patients from our Berlin and Lyon cohorts (Fig. 10a); Control: age-matched subset (n=2745) of a previously published cohort of children in the same area of Germany(Beer 2017). EBV serology was determined in-hospital (VCA and/or EBNA1) or by EBNA1 ELISA. The difference between seropositive and seronegative individuals in both cohorts was compared. **b** LCL from healthy donors (n=6) were treated with TGF-β, or with serum from MIS-C (n=5) or severe COVID-19 (n=6), with or without pre-incubation with TGF-β1,2,3 blocking antibodies. EBV reactivation was evaluated by qPCR by quantifying the reactivation transcription factor *BZLF1.* Results were calculated using the 2^{-ΔCt} method. P-values were calculated by **a** one-tailed Fisher's exact test to test if seroprevalences were increased or **b** paired two-tailed Mann-Whitney U-tests.

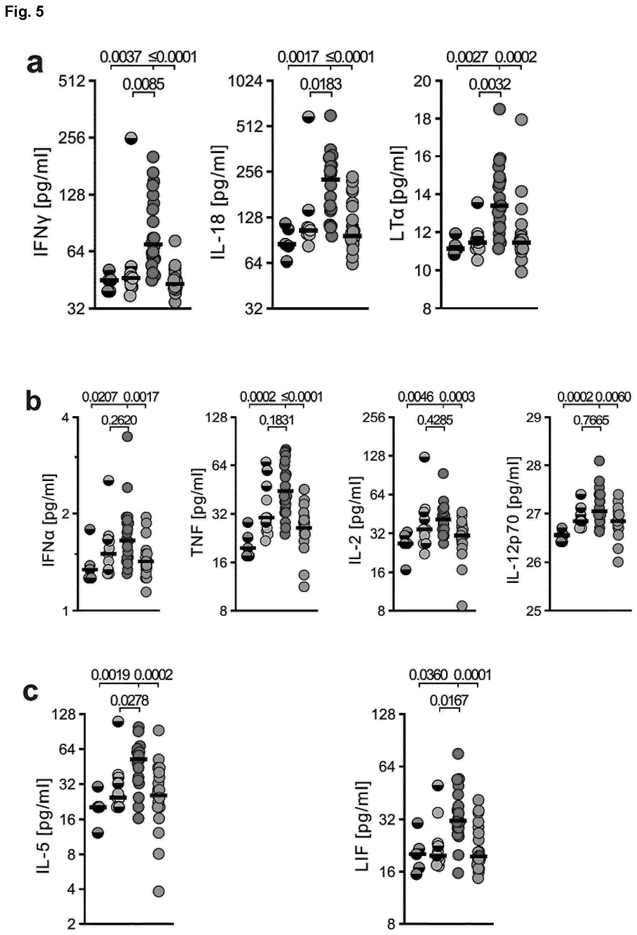

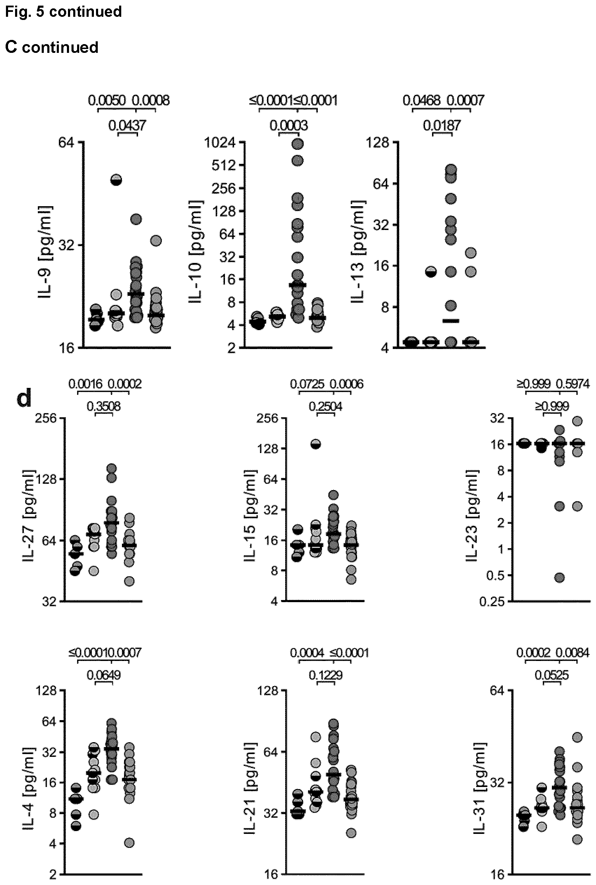

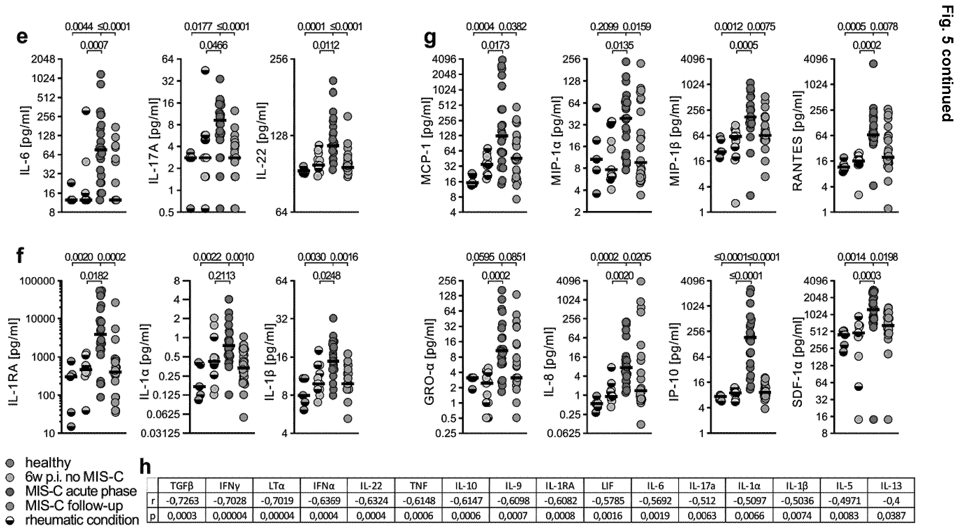

**Figure 5****: Multiplex cytokine profiling in MIS-C. a-f** cytokine and chemokine profiles of serum from MIS-C patients during the first 7 days of hospitalization (n=22) and at follow-up visits (n=28 time points, 23 patients), of not infected children (n=5) and of children 6 weeks after MIS-C, that did not develop MIS-C (n=11 of which some have an underlying rheumatic condition n=5). **a** significantly **b** not-significantly upregulated Type 1 cytokines. **c** significantly d not-significantly upregulated Type 2 cytokines. **e** Type 3 cytokines. **f** IL-1 family cytokines and **g** chemokines. **h** correlation of cytokine quantity versus time after hospitalization and initiation of treatment. **a-g** a non-parametric ANOVA (Kruskal-Wallis test) was used followed by a Dunn's multiple comparison test with correction for multiple comparisons, or **h** a spearman-correlation was used. Data for healthy individuals is depicted on the very left of each plot, followed by data (shown at second from left in each plot) for children 6 weeks after MIS-C, that did not develop MIS-C, wherein the data shown second from the right of each plot relates to MIS-C patients during the first 7 days of hospitalization (`acute phase'), the data at the far right of each plot relates to patients at follow-up visits.

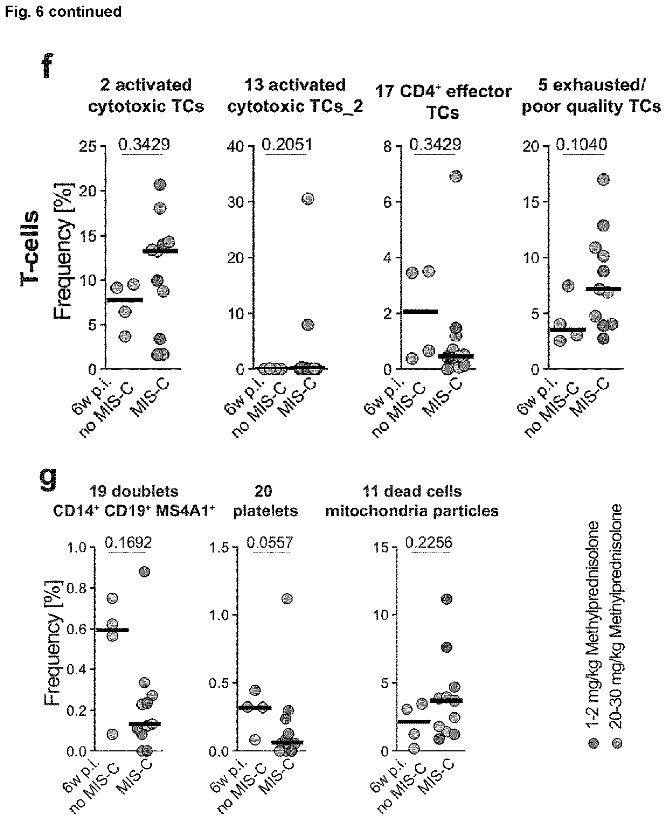

**Figure 6****: Single cell sequencing in MIS-C. a** schematic outline of the scRNAseq approach, **b** flow cytometry gating strategy. c UMAPs separated by individual patients organised by duration and dosage of methylprednisolone treatment. B07 (MIS-C patient Berlin 07) , B09 and B 10 were captured before treatment with methylprednisolone, B08 was treated with 1-2mg/kg methylprednisolone for 2 days, B06 was treated with 1-2 mg/kg methylprednisolone for 3 days. Patients B01, B02 B04, B05 and B11 were treated with 20-30 mg/kg methylprednisolone for 1 day and B03 was treated with 20-30 mg/kg methylprednisolone for 4 days before capturing (Extended data Table6). Paediatric controls 6 weeks p.i. with SARS-CoV-2 (ctrl 1-4) did not receive medication. **d-g** Frequencies of cells per cluster in both groups for clusters not included in Figure 1. Treatment with methylprednisolone is colour (greyscale)-coded. The data depicted on the right of each plot relates to treatment with either 1-2 mg/kg or 20-30 mg/kg methylprednisolone. p values were determined by two-tailed Mann-Whitney U-tests.

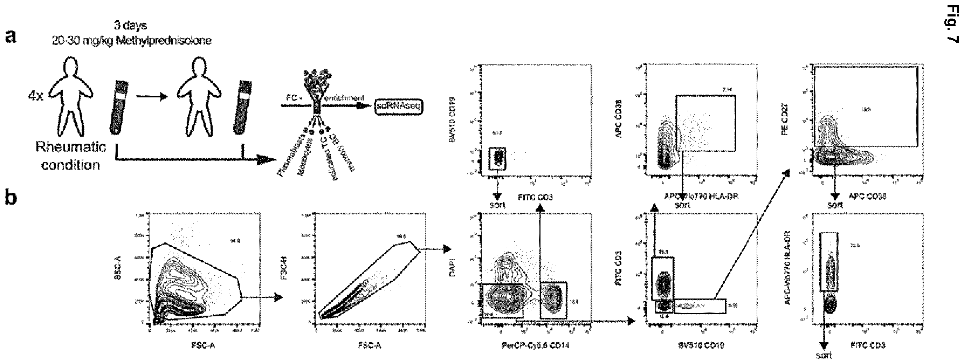

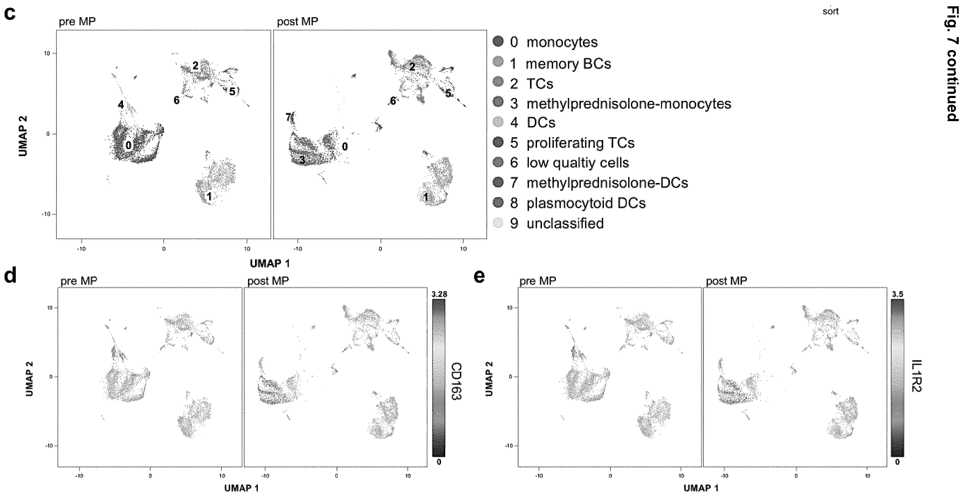

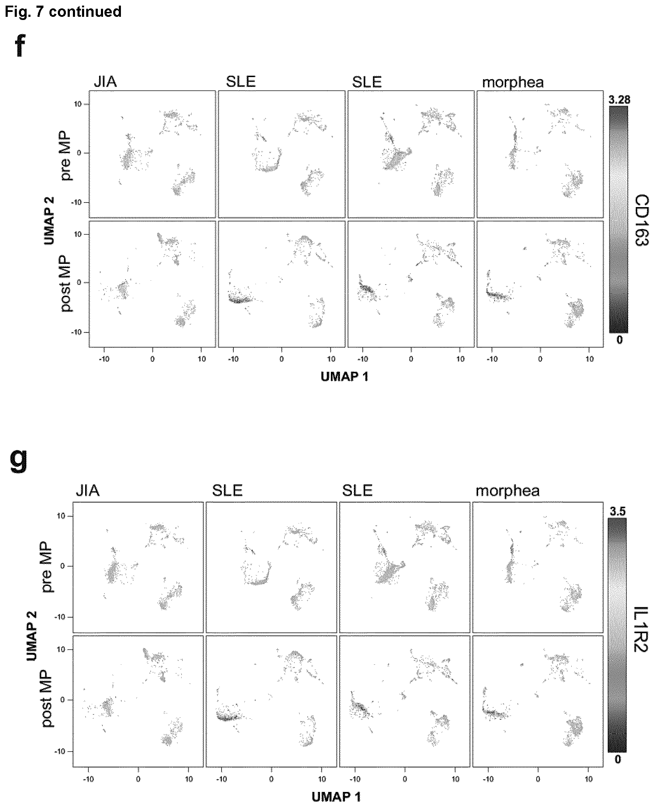

**Figure 7****: Single cell sequencing of methylprednisolone treated patients cells,** a schematic outline of the scRNAseq approach to identify the effect of methylprednisolone treatment. **b** gating strategy used for scRNAseq experiment. **c** UMAP of a total of 13646 cells enriched by FACS for monocytes, HLA-DR^{high} cells, CD38⁺ T-cells and CD27⁺ B-cells of n=4 patients prior (pre MP, 6839 cells) and after (post MP, 6807 cells) a three-day treatment with 20-30mg/kg/day methylprednisolone. 1 patient was diagnosed with juvenile idiopathic arthritis (JIA), 2 patients with systemic lupus erythematosus (SLE) and 1 patient with morphea. **d** CD163 expression and **e** IL1R2 which defines cluster 3 and 18 of the MIS-C cohort is depicted separately for pre MP and post MP. **f-g** UMAP separated for individual patients' cells before and after treatment are depicted with **f** CD163 expression and **g** IL1R2 expression depicted separately.

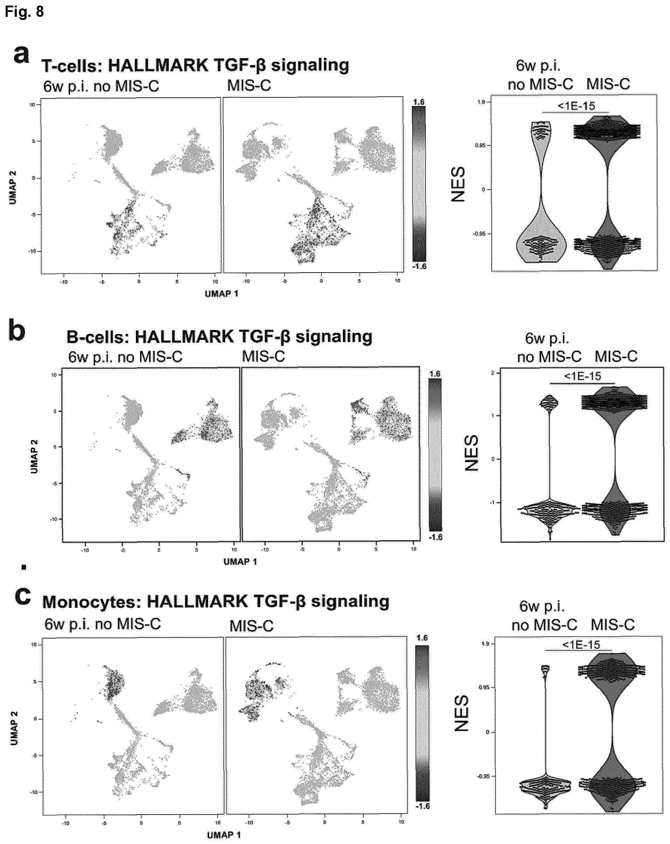

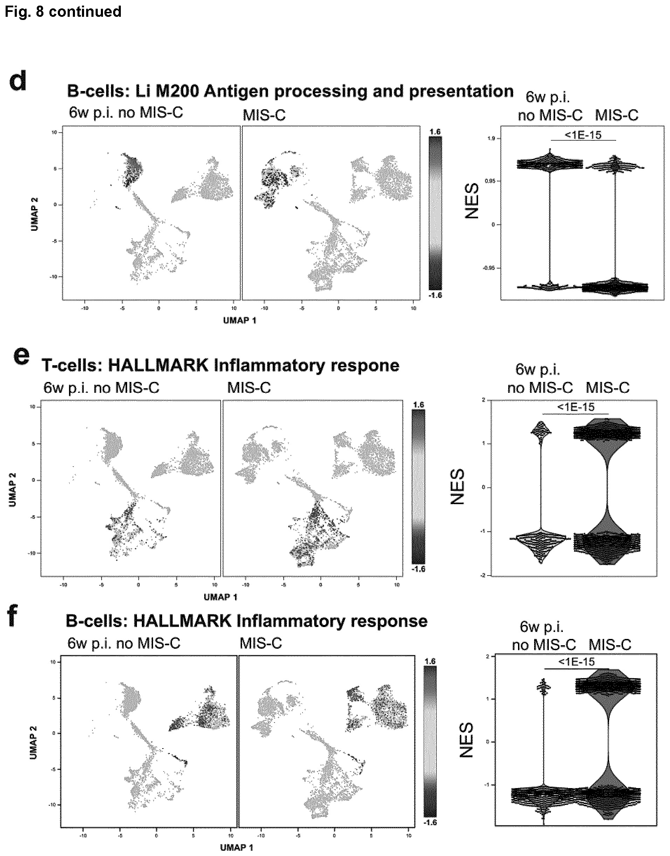

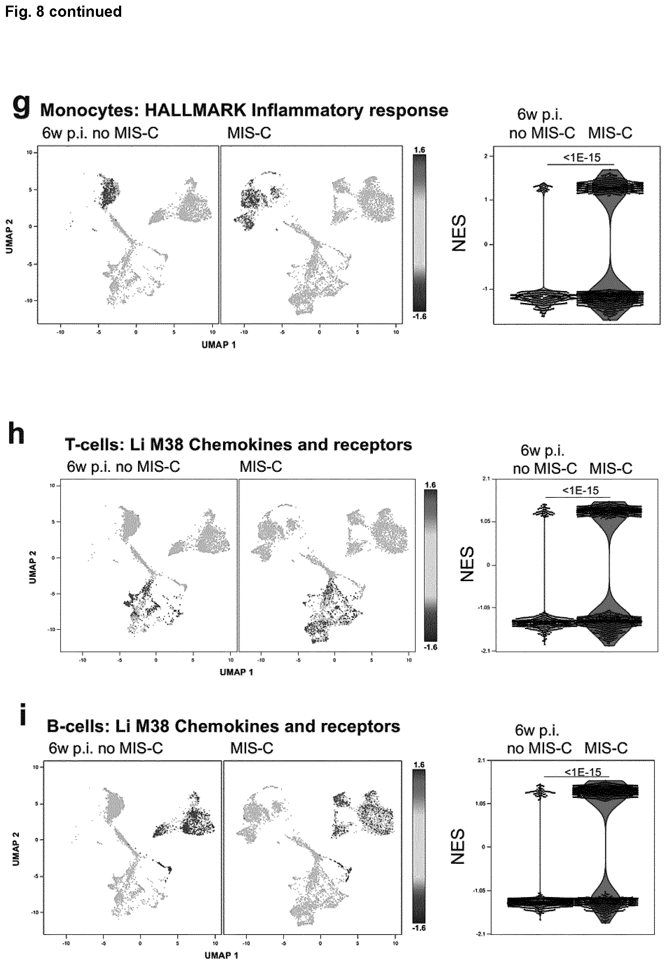

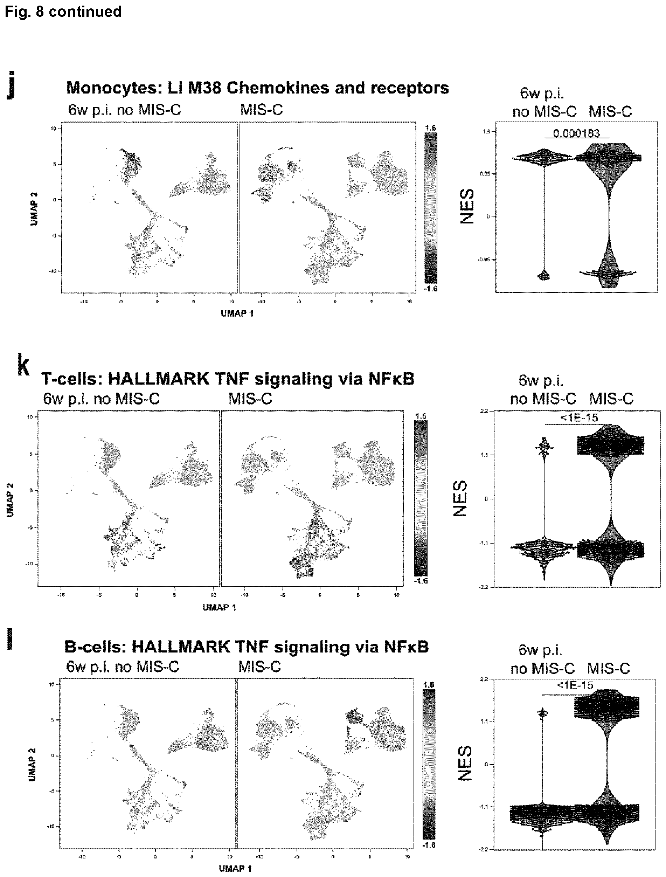

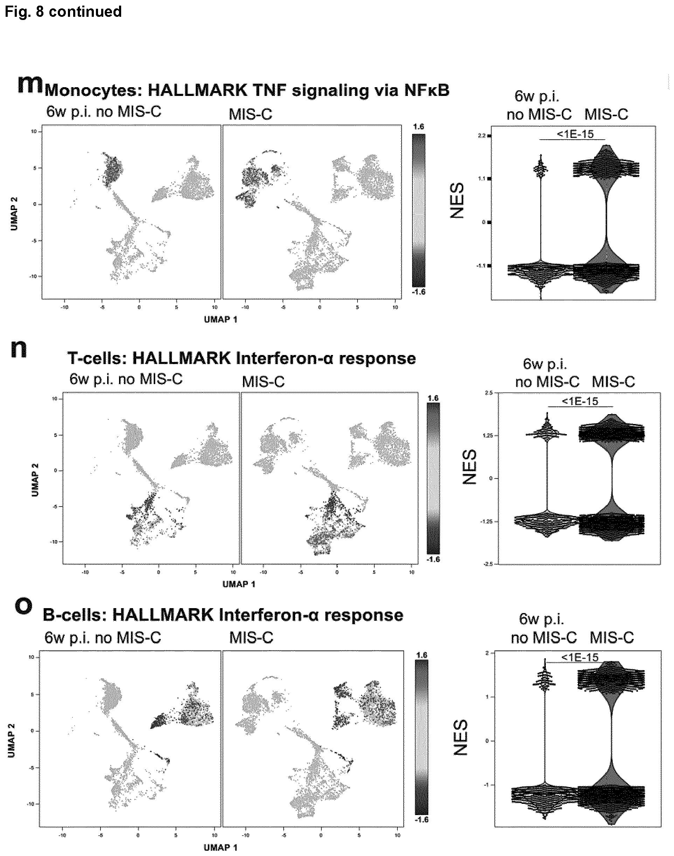

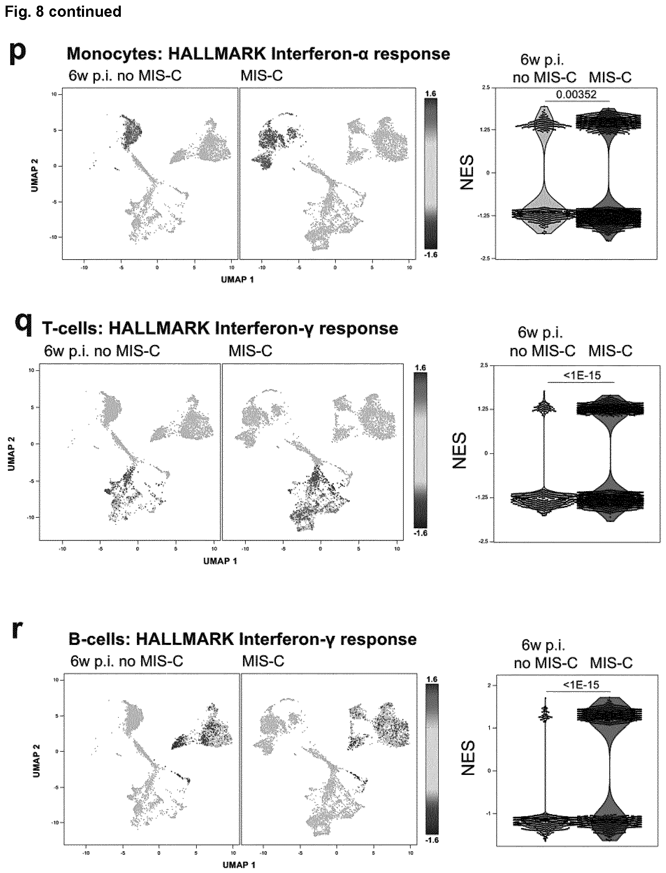

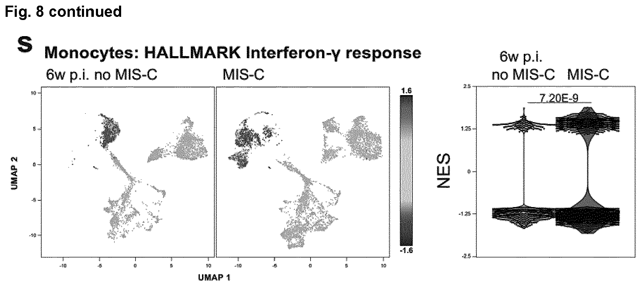

**Figure 8****: Gene Set Enrichment Analysis on a single cell level in MIS-C. a-s** GSEA for indicated gene sets on indicated cell populations. For each GSEA the normalized enrichment score (NES) is depicted on the rarefied UMAP (see Figure 1c). The NES of all cells with significant positive or negative enrichment are plotted in a violin-plot. p values are determined by two-tailed Fisher's exact test testing for positive versus negative enrichment.
**Figure 9****: TGF-β impairs T-cell reactivity to viral epitopes. a** gating strategy used in flow cytometry of the T-cell reactivity assays depicted in Figure 2. Cells were identified by size and granularity in a FSC-vs SSC plot, followed by doublet exclusion in an FSC-A vs. FSC-H plot. Dump (DAPI, CD14 and CD19)⁺ cells were also excluded. As CD3 is downregulated after T-cell activation (SEB plot in second row), the gate was extended to include CD3^{low} CD45Ro⁺ cells. CD4⁺ epitope-specific T-cells were identified as CD69⁺ CD154⁺ and CD8⁺ epitope specific T-cells were identified as CD69⁺ CD137⁺. SEB was used as a positive control for correct gating. **b** Frequencies of antigen-specific reactivation and overall activation of CD4⁺ and **c** CD8⁺ T-cells from healthy donors (n=6) treated with 50 ng/ml TGF-β1. p values for **b-c** were determined by paired two-tailed Mann-Whitney U-tests.

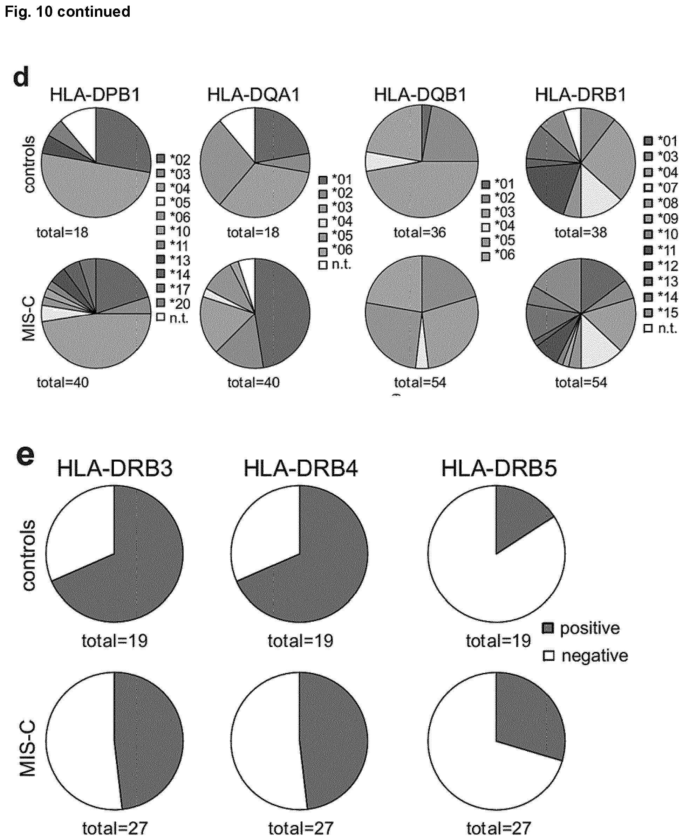

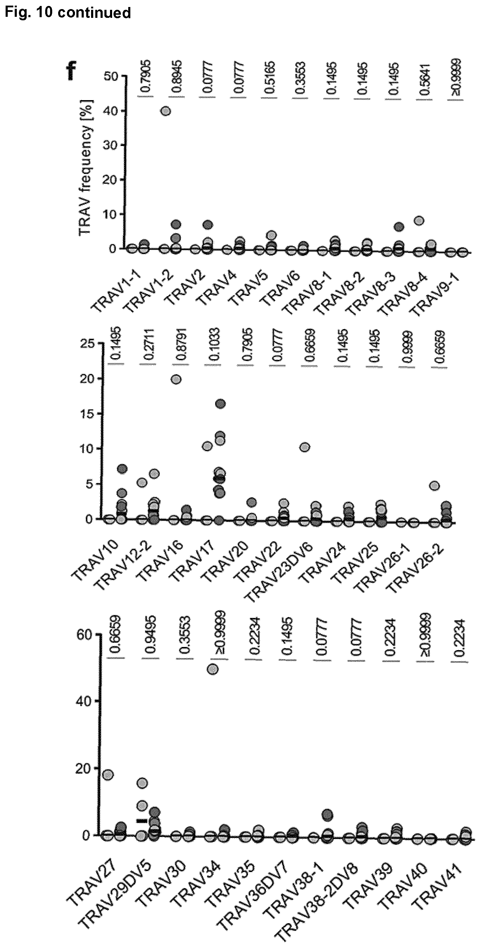

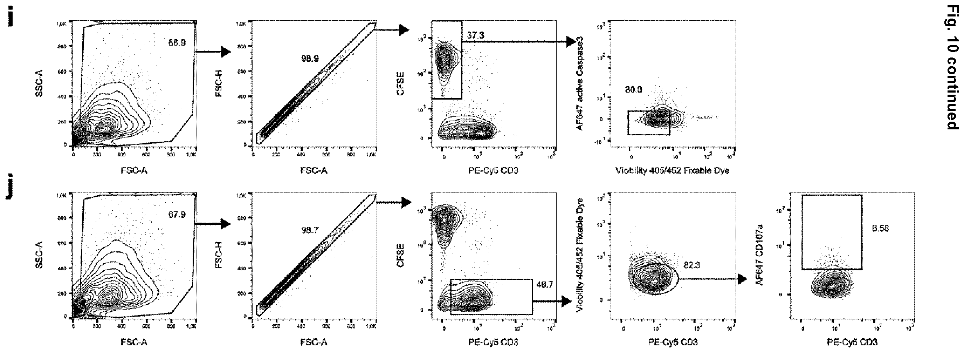

**Figure 10****: Altered TCR repertoires in MIS-C are accompanied by specific HLA-haplotypes. a** flow cytometric analysis of TCRVβ21.3+ T-cells for CD4⁺ or CD8⁺ T-cells from patients with MIS-C before start of treatment (untreated, n=9), after 1-3 days of 1-2 mg/kg methylprednisolone (1-2mg/kg MP, n=2) or after 1-3 days of 20-30 mg/kg methylprednisolone (20-30mg/kg MP n=10). **b** significantly regulated TRBV determined by TCR sequencing of activated T-cells. Dots indicate the frequency of specific TRBV in each sample relative to all TCRs sequenced. Healthy individuals showed on average higher TRBV frequency than children 6 weeks after MIS-C, that did not develop MIS-C, and subjects receiving treatment with either 1-2 mg/kg or 20-30 mg/kg methylprednisolone. **c** HLA-class I haplotyping and **d-e** HLA-class-II haplotyping of our MIS-C cohort (n=20 patients and n=10 healthy controls including the 4 children used as a control for the scRNAseq experiments). Additionally, HLA-haplotyping from a previously published MIS-C cohort (n=7 patients and 9 controls)¹⁵ was included. **f-i** Flow cytometry gating strategies used for **f** sorting of T-cells obtained after the ARTE-assay and used for generation of a virus-specific TCR library (Figure 3e-f) **g** for sorting of TCRVβ21.3-positive or -negative CD4⁺ or CD8⁺ T-cells for co-culture experiments (Figure 3g), **h** analysing viable EBV infected B cells after co-culture with T-cells for Figure 3h-i and **i** analysing CD107a+ T-cells after co-culture with EBV-infected B-cells (Figure 3h-i). p values **f**or **a** indicate contingencies of oligoclonal T-cell expansion above the laboratory determined upper cut-off and were determined by two-tailed Fisher's exact test. For **b** p values were calculated by two-tailed Mann-Whitney U-tests.

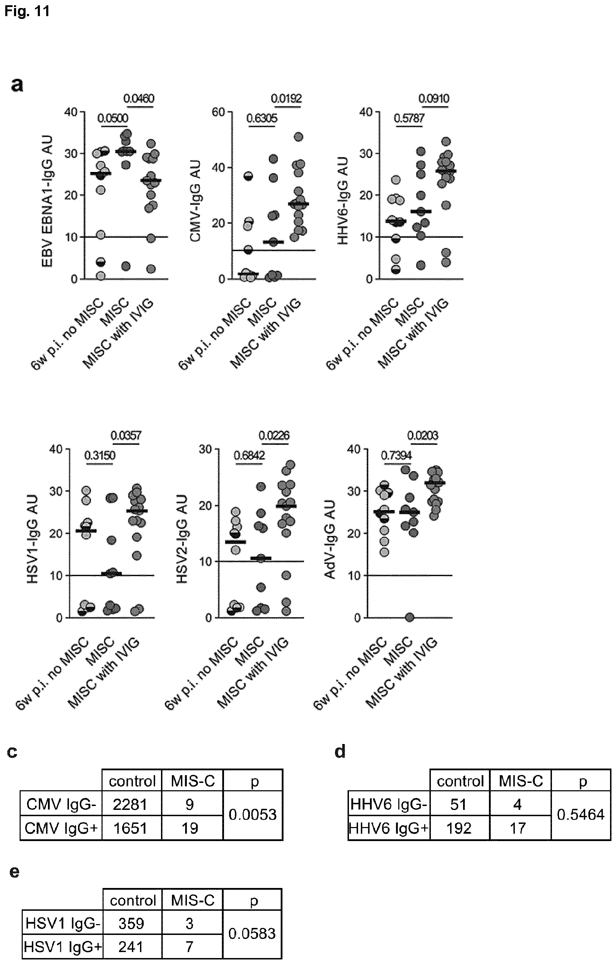

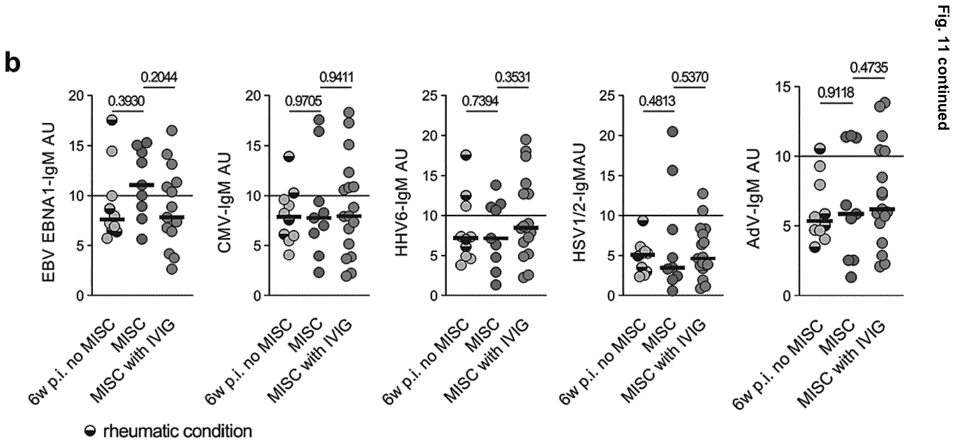

**Figure 11****: Latent virus seroprevalences in MIS-C. a** Semi-quantitative analysis of the indicated virus-specific IgG or **b** IgM present in the serum of paediatric controls 6 weeks p.i. who did not develop MIS-C (n=10), MIS-C patients before treatment with IVIG (n=10), and after treatment with IVIG (n=15). Quantification was made by ELISA. The cut-off was determined by assay specific controls and is set to 10 arbitrary units (AU). **c-e** Seroprevalences were compared to previously published German patient cohorts c for CMV⁶⁶, **d** for HHV6⁶⁸ and **e** for HSV1⁶⁷. p values for **a-b** are determined by two-sided Mann-Whitney U-tests and for **c-e** by one-tailed Fisher's exact test to test if seroprevalences are increased.
**Figure 12****:** Hypothesized mechanism of MIS-C pathogenesis: a combination of genetic susceptibility and reactivation of dormant latent virus infection (frequently with EBV) are required for MIS-C development. In individuals with this combined susceptibility an infection with SARS-CoV-2 triggers latent virus reactivation and induces a prolonged and excessive TGF-β response. The high TGF-β levels lead to a continued EBV reactivation and reduced functionality of antigen-specific T-cells. The resulting viremia leads to oligoclonal expansion of the dysfunctional T-cells, which are incapable of controlling the virus, resulting in a viral sepsis-like hyperinflammation.

### EXAMPLES

The invention is further described by the following examples. These are not intended to limit the scope of the invention but represent preferred embodiments of aspects of the invention provided for greater illustration of the invention described herein.

### Material and Methods

### Human participants

Thirty-nine patients were recruited from the Charité Universitätsmedizin Berlin Department of Pediatric Respiratory Medicine, Immunology and Critical Care Medicine. MIS-C was diagnosed in all patients by a consultant paediatric rheumatologist according to the definitions from WHO(Penner, Abdel-Mannan et al. 2021), CDC(CDC) and RCPCH(RCPCH 2020). Blood samples were taken from the acute phase of MIS-C and during follow-up visits in the out-patient clinic. Paediatric controls included in this study had a positive SARS-CoV-2-PCR result 6 weeks before inclusion. Patient characteristics are summarized in. This study was approved by the Institutional Review Board of the Charité (Pa-COVID19 and EA2/178/22) and the Comit6 de Protection des Personnes Sud Méditerranée I, Marseille, France) (ID-RCB: 2020-A01102-37) for the French patients. Written and informed consent was provided by all legal representatives of the patients that participated in this study.

### Isolation of PBMC and serum

Peripheral blood was drawn into EDTA collection tubes and SST^{™} tubes as previously described(Witkowski, Tizian et al. 2021). PBMC were isolated from peripheral blood by Ficoll-Paque^{™} PLUS (Cytiva) density gradient centrifugation at room temperature. Cells were either used directly for analysis or stored at -80 °C in heat-inactivated foetal bovine serum (FCS; Corning, 35-079-CV) with 10% v/v dimethylsulfoxide before analysis. Serum samples were stored at -80 °C before analysis.

### Cytokine measurements

A bead-based multiplex cytokine array (Cytokine/Chemokine/Growth Factor 45-Plex Human ProcartaPlex^{™} Panel1, ThermoFisher Scientific) was used according to the manufacturer's protocol. For TGF-β measurements, TGF-β was transformed to the bioactive form using 1 N HCL followed by neutralisation with 1.2 N NaOH. A Human TGF-β1 Simplex ProcartaPlex kit (Thermo Fisher Scientific) was used as previously described(Witkowski, Tizian et al. 2021).

### Cell isolation from PBMC for single-cell sequencing

Frozen PBMC from 11 MIS-C patients and from 4 patients who were 6 weeks post-infection with SARS-CoV-2 without developing MIS-C were thawed in RPMI 1640 (Gibco 61870-044) with 20%v/v FCS (FCS; Corning, 35-079-CV). Cells were washed once in PBS (Th. Geyer GmbH & Co. KG) containing 1%w/v BSA (PAN Biotech GmbH P06-1391500), 2mM EDTA (Invitrogen) and 2µg/ml Actinomycin D (Sigma-Aldrich). Cells were incubated with human FcR blocking reagent (1:50; Miltenyi Biotec) and stained using one of four TotalSeq^{™} anti-human Hashtags (C0251 C0252, C0253 or C0254) (1:250, Clone LNH-94 + 2M2, BioLegend) , FITC anti-CD3 (1:100, UCHT1, in house), PerCP-Cy5.5 anti-CD14 (1:50, TÜK4, BioLgend), APC anti-CD38 (1:25, HIT2, Biolegend), APC-Vio770 anti-HLA-DR (1:100, AC122, Miltenyi Biotec), V500 anti-CD19 (1:200, HIB19, BD Biosciences) and PE anti-CD27 (1:100, M-T271, BioLegend) at 4°C for 30 minutes. Directly prior to sorting on a BD Ariall (BD Biosciences), DAPI (Sigma-Aldrich, 0,1 µg/ml) was added.

Activated T-cells were identified as DAPI⁻, CD14⁻, CD19⁻, CD3⁺, CD38⁺ and HLA-DR^{high}, memory B-cells were identified as DAPI⁻, CD14⁻, CD3⁻, CD19⁺, CD27⁺ (and CD38^{high} for plasmablasts), and monocytes were identified as DAPI⁻, CD14⁺ (Data Fig. 6b).

For identifying methylprednisolone effects on activated cells, cells were sorted on a MA900 Multi-Application Cell Sorter (Sony Biotechnology). Instead of plasmablasts, lineage⁻ HLA-DR^{high} cells were sorted (identified as DAPI⁻, CD14⁻, CD19⁻, CD3⁻, and HLA-DR^{high}) (Fig. 7b). Sorted cells were counted using a MACSQuant Analyzer 16 (Miltenyi Biotec) and processed for scRNA-seq using the 10X Genomics technology.

### Single-cell RNA library preparation and sequencing

Single-cell RNA library construction and sequencing was done as previously described(Ferreira-Gomes, Kruglov et al. 2021, Witkowski, Tizian et al. 2021). Briefly, Chromium Next GEM Single Cell 5' reagent kits v2 (dual index) with feature barcode technology for cell surface protein (CITE) mapping (10X Genomics) were used according to the manufacturer's protocol. Final CITE-Seq libraries were generated after index PCR with dual Index Kit TN Set A (10X Genomics) while final GEX and TCR/BCR libraries were generated after fragmentation, adapter ligation and final index PCR with a dual Index Kit TT Set A (10X Genomics). Libraries were quantified using a Quibit HS DNA assay kit (LifeTechnologies) and fragment sizes were determined using a HS NGS Fragment (1-6000 bp) kit (Agilent).

Sequencing was performed on a NextSeq2000 sequencer (Illumina) using a P3 reagent cartridge (100 cycles) (Illumina) with the following recommended sequencing conditions: read1: 26 nt, index1: 10 nt, read2: 90 nt, index2: 10 nt.

### Single-cell transcriptome analysis

Raw sequence reads were processed using cellranger (version 5.0.0), including the default detection of intact cells based on the EmptyDrops method(Lun, Riesenfeld et al. 2019). Demultiplexing, mapping, detection of intact cells, as well as quantification of gene expression was performed using cellranger's count pipeline in default parameter settings with refdata-cellranger-hg19-1.2.0 as reference, Hashtag 1-4 as feature reference and expected number of 3000 cells per sample. Of note, the used reference does not contain immunoglobulin genes and TCR genes as defined by the respective biotype, expect for *TRBV11-2* which was removed in addition. This led to 8531, 4356 and 7644 intact cells for MIS-C pools and 8060 for controls. Next, cellranger's aggr was used to merge the libraries without size normalisation and further analysed in R (version 4.1.2) using the Seurat package (version 4.0.5)(Hao, Hao et al. 2021). In particular, the transcriptome profiles were read using Read10x and CreateSeuratObject and log-normalised using NormalizeData. A uniform manifold approximation and projection (UMAP) was computed using ScaleData, RunPCA to compute 50 principle components and RunUMAP using 1:50 dimensions. Transcriptionally similar clusters were identified by shared nearest neighbor (SNN) modularity optimisation using FindNeighbors with pca as reduction and 1:50 dimensions as well as FindClusters with resolutions ranging from 0.1 to 1.0 in 0.1 increments using FindCluster. By visual inspection of the percentage of mitochondrial genes, UMI counts, number of identified genes as well as expression of typical marker genes projected on the UMAP, clustering with a resolution of 0.6 was judged to best reflect the transcriptional community structure. Clusters comprising low quality cells as well as clusters comprising contaminations were not considered in further analyses. Samples were demultiplexed using hashtag reads after log-normalization and manual distinction of positively tagged cells using histograms.

### Single-cell immune profiling

T-cell receptor annotations were based on single cell immune profiling. In particular, raw sequence reads were processed by cellranger (version 5.0.0) with vdj in default parameter settings for demultiplexing and assembly of the TCR sequences using refdata-cellranger-vdj-GRCh38-alts-ensembl-2.0.0 as reference. Annotations of the TCR were assigned to the corresponding cells in the single cell transcriptome analysis by identical cellular barcodes. In case of multiple contigs, the most abundant, productive and fully sequenced contig for the alpha and beta or gamma and delta chain respectively. Noteworthy, cellranger mislabelled *TRBV11-2* with *TRBV11-1* annotations as judged by multiple alignment of the sequences of the respective contigs to the references of *TRBV11-1* and *TRBV11-2* as well as mapping of transcriptome to the *TRBV11-2* gene locus.

### Gene Set Enrichment Analysis

A Gene Set Enrichment Analysis (GSEA) was performed based on the difference to the mean of log normalised expression values of monocytes, B-cells including plasmablasts, or T-cells manually selected using cloupe (version 6.3.0) in the analysed set as pre-ranked list and 1000 randomisations(Mootha, Lindgren et al. 2003, Subramanian, Tamayo et al. 2005). The GSEA was performed separately for T-cells, B-cells (with plasmablast

s) and monocytes. Significant up- or downregulation was defined by a FDR ≤ 0.50 and normalised p-value < 0.05. For visualisation, NES for significant cells were plotted. The GSEA was performed for indicated cells using hallmark gene sets(Liberzon, Birger et al. 2015), our previously published TGF-β NK-cell gene set(Witkowski, Tizian et al. 2021) and previously defined immune transcription modules(Li, Rouphael et al. 2014). Hallmark gene sets were obtained from the MSigDB Collections(Liberzon, Birger et al. 2015). The GSEA results were visualised by projection of the NES scores on UMAPs as well as on violin plots of the NES score of significant enriched cells, i.e. positive NES score.

### Ex vivo stimulation of isolated mononuclear cells

Memory T-cell restimulation experiments were performed in RPMI 1640 medium (Miltenyi Biotec), supplemented with 5% v/v human AB serum (Sigma-Aldrich) or 10% v/v patients' sera, 100 U/ml Penicillin-Streptomycin (Gibco) and 2 mM L-glutamine (HyClone). For antigen-reactivity measurements, a total of 5×10⁵ PBMC were stimulated for 16 h with the following antigens for PepTivator^{®} SARS-CoV-2 Prot_N, PepTivator^{®} SARS-CoV-2 Prot_S, PepTivator^{®} SARS-CoV-2 Prot_M, omicron specific pepTivator SARS-CoV-2 Prot_S B.1.1.529 (all 60 nM/peptide; Miltenyi Biotech), for EBV: PepTivator^{®}EBV Consensus, premium grade (60nmol/peptide; Miltenyi Biotech); for HSV1: PepTivator^{®}HHV1 Envelope Glycoprotein D, research grade (60nmol/peptide; Miltenyi biotech), for HSV2: PepMix^{™} HSV2 (gD) (15 nM/peptide; JPT peptide technology); for HHV6 (U54) Peptide Pool (15 nM/peptide; peptides&elephants), for adenovirus (AdV): PepTivator^{®}AdV Select (60 nM/peptide; Miltenyi Biotech), staphylococcus enterotoxin B (SEB) 1µg/ml or peptide pools of cytomegalovirus (CMV) pp66 (60nM/peptide; Miltenyi Biotech) in presence of 1 µg/mL CD40 and 1 µg/mL CD28 functional grade pure Ab. When used, patients' sera were pre-incubated with antibodies directed against TGF-β1, TGF-β2 and TGF-β3 (50µg/ml, R&D Systems, MAB1835-SP) for 10 minutes.

Cells were incubated with FcR blocking reagent (1:50, Miltenyi Biotec) and stained for flow cytometric analysis with CD14 VioBlue (1:200, REA599, Milenyti Biotec), CD19 VioBlue (1:100, REA675, Miltenyi Biotec), CD45Ro BV510 (1:50, UCHL1, BD Biosciences), CD3 PE Cy5 (1:200, UCHT1, BioLegend), CD8a BV650 (1:100, RPA-T8, BioLegend), CD4 PerCPeFlour710 (1:200, SK3, eBioscience), CD69 APC-Cy7 (1:100, FN50, BioLegend), CD154 PE (1:100, REA238, Miltenyi Biotec) CD137 PE Cy7 (1:100, 4B4-1, BioLegend) as mentioned above. Directly prior to analysing on a MACSQuant Analyzer 16 (Miltenyi Biotec), DAPI (Sigma-Aldrich, 0,1pg/ml) was added.

### HLA-Haplotyping

DNA was extracted from whole blood or by buccal swaps (eSwab, Copan) using the QIAamp^{®} DNA Micro kit (QIAGEN). The DNA was subjected to HLA genotyping for HLA-A, B, C, DRB1, DRB3, DRB4, DRB5, DQA1, DQB1 and DPB1. HLA typing was performed using the Protrans NGS for 7 Loci kit (Protrans Medical Diagnostics) by next generation sequencing (NGS). The isolated genomic DNA was used in eight multiplex PCR reactions and labelled with Nextera XT index primer kits (Illumina). After group-specific pooling, followed by CleanPCR Beads purification (Beckman Coulter Inc.) and normalization (Quant-iT PicoGreen, Thermo Fischer Scientific), a MiSEQ library was prepared and sequenced on an Illumina MiSeq platform. HiType software (Inno-Train) based on the most recent HLA database was used for data analysis.

### TCR-library preparation of virus-specific T-cells by antigen-reactive T-cell enrichment assay

At least 1×10⁷ total PBMC were stimulated as mentioned above. After stimulation, cells were stained with TotalSeq^{™} anti-human Hashtags as previously mentioned, followed by CD154 MACS enrichment according to the manufacturer's protocol (CD154 MicroBead Kit, human; Miltenyi Biotec). After MACS, cells were incubated with human FcR blocking reagent (1:50, Miltenyi Biotec) and stained for FACS enrichment with PE-anti Biotin (1:100, Bio3-18E7, Miltenyi Biotec), CD154 PE (1:40, REA238, Miltenyi Biotec), CD14 VioBlue (1:200, REA599, Milenyti Biotec), CD19 VioBlue (1:100, REA675, Miltenyi Biotec), CD3 PE Cy5 (1:200, UCHT1, BioLegend) and CD69 APC (1:100, REA824, Miltenyi Biotec). DAPI (Sigma-Aldrich, 0,1 µg/ml) was added and cells were sorted on a MA900 Multi-Application Cell Sorter (Sony Biotechnology) according to scatter properties, viability (DAPI⁻) and dump⁻ (CD14 and CD19) specifically for CD3⁺, CD154⁺ and CD69⁺ antigen-specific cells (Extended Fig. 6f). A heat map was generated using the R package pheatmap.

### EBV-killing assay

EBV-immortalised B-cells (LCL) from healthy donors were generated as previously described(Moosmann, Khan et al. 2002). From the same donors, T-cells were isolated from PBMC using the Human Pan T-Cell Isolation Kit (Miltenyi Biotec), and incubated with human FcR-blocking reagent (1:50, Miltenyi Biotec) and stained with CD3 FITC (1:100, UCHT1, in-house), CD8a BV650 (1:100, RPA-T8, BioLegend), CD4 PerCPeFlour710 (1:200, SK3, eBioscience), TCR Vβ21.3 APCVio770 (1:100, REA894, Miltenyi Biotec). Prior to sorting, DAPI (Sigma-Aldrich, 0,1pg/ml) was added. Cells were sorted on a MA900 Multi-Application Cell Sorter (Sony Biotechnology). Viable (DAPI⁻) and VioBlue⁻, CD3⁺ cells were further sorted according to CD4, CD8 and TCR Vβ21.3 expression (Extended Fig 6g). 4 × 10⁴ cells were seeded and polyclonally expanded using Dynabeads^{™} human T-Activator CD3/CD28 (ThermoFisher) at a bead-to-cell ratio of 1:1. For CD4 cells, 50 U/ml human recombinant IL-2 (Peprotech) was added and for CD8 expansion, 10 ng/ml IL-15 (Peprotech) was added to the medium (TexMACS Medium (Miltenyi Biotec) with 5% human AB serum (Sigma-Aldrich) and 100 U/ml Penicillin/Streptomycin (Gibco)). LCL were marked with CFSE (1:2000, BioLegend). After expansion, viable, CFSE-marked LCL and expanded T-cells from each donor were counted after staining with DAPI and 1×10⁴ viable cells were seeded with respective numbers of viable T-cells and centrifuged for 2 minutes at 200 rpm. LCL were co-cultured for 4 h with CD8⁺ T-cells and 24 h with CD4⁺ T-cells at 37°C 5% CO₂. For the CD107a assay, CD107a AlexaFlour647 (1:3000, H4A3, BioLegend) and Brefeldin A (5µg/ml, BioLegend) was added for the last 4 hours. Cells were stained with fixable-viability dye ZombieAqua (1:400, BioLegend) or Viobility 405/452 Fixable Dye (1:100, Miltenyi Biotec), CD3 PE Cy5 (1:200, UCHT1, BioLegend), CD8a BV650 (1:100, RPA-T8, BioLegend), CD4 PerCPeFlour710 (1:200, SK3, eBioscience), TCR Vβ21.3 APCVio770 (1:100, REA894, Miltenyi Biotec). Afterwards, cells were fixated and permeabilised with the True Nuclear staining kit (BioLegend) and stained with active caspase-3 AlexaFlour647 (1:50, BD Bioscience). Viable LCL were defined as CFSE⁺, fixable viability dye⁻ and active caspase-3⁻ cells (Extended Fig 6h). Cell counts were normalised to LCL cultured without T-cells and CD107a (Extended Fig 6i) staining was normalised to T-cells cultured without LCL.

### ELISA

Serum IgG-antibody levels against HSV1 (Abnova, KA0229), HSV2 (Abnova, KA0231), EBNA1 (abcam, ab108731), CMV (Abnova, KA1452), HHV6 (Abnova, KA1457) and Adenovirus (Creative Diagnostics, DEIA2382) and serum IgM antibody levels against HSV1/2 (Abnova, KA4842), EBNA1 (Abnova, KA1449), CMV (Abnova, KA0228), HHV-6 (Creative Diagnostics, DEIABL57) and Adenovirus (Creative Diagnostics, DEIA1767) were measured in first serum samples obtained from patients according to manufacturer's manuals. Arbitrary units were calculated based on reference controls.

### EBV transcript detection in single cell dataset

For detection of EBV reactivation, unmapped reads from the transcriptome analysis were extracted from the bam files from the transcriptome analysis and mapped to the human gammaherpesvirus 4 references (NCBI Assemblies:GCF_002402265.1, GCF_000872045.1.). Specific binding as well as correct gene annotation was verified using blast(Altschul, Gish et al. 1990, Sayers, Bolton et al. 2022) with standard databases. EBV-UMI counts were assigned to the corresponding cells in the single cell transcriptome analysis by identical cellular barcodes.

### EBV-reactivation assay

For EBV-reactivation, LCL were incubated in RPMI 1640 (Gibco 61870-044) with 10% v/v FCS (Biowest S1600-500) or 10% v/v patient's serum and 1% v/v Penicillin/Streptomycin (Gibco 15140-122). 10 ng/ml rh-TGF-β (PeproTech, 100-21) was used as a positive control. If TGF-β was neutralised, the patient's sera was pre-incubated for 10 minutes with 50µg/ml of antibodies directed against TGF-β1, TGF-β2 and TGF-β3 (R&D Systems, MAB1835-SP). Cells were harvested after 24 hours and total RNA was extracted using the RNeasy^{®} Plus Micro Kit (Qiagen). cDNA was transcribed using the TaqMan reverse transcription kit using random hexamers (Life Technologies). TaqMan^{®} PCR was performed using human *HPRT1* primers and probes of TaqMan^{®} gene expression assays (Life Technologies) as well as the following combination of primers and probes for the transcription factor *BZLF1,* which induces the lytic EBV-replication cycle, forward: 5'-CTCAACCTGGAGACAATTCTACTGT-3', reverse: 5'-TGCTAGCTGTTGTCCTTGGTTAG-3', probe: 5'FAM-CTGCTGCTGCTGTTTG-3'NFQ (Life Technologies). Samples were measured on a QuantStudio^{™} 5 Real-Time PCR System (Applied Biosystems).

### Statistical analysis and reproducibility

GraphPad Prism v8.00 for Windows (GraphPad Software) was used for statistical analysis of the data. If applicable, all data are plotted as individual values and the median is given as data summary. For two group comparisons of non-normal distributed data, a two-tailed Mann-Whitney-Test was used. If normal distribution can be assumed a two-tailed t-test was used. For paired samples, the paired-test equivalence was used. For multiple group comparisons, two-tailed non-parametric ANOVA (Kruskal-Wallis test) was used, followed by a Dunn's multiple comparison test with correction for multiple comparisons. Correlations were calculated using a Spearman-Correlation. Testing for significance in Gene Set Enrichment Analysis: if only positive enrichment was quantified, a two-tailed-Mann-Whitney-Test was used, otherwise, to test for positive and negative enrichment in bimodal data a two-tailed Fisher's exact test was used. Testing for increased seroprevalences of latent virus infections: a one-tailed Fisher's exact test was used. The significance threshold for all tests was set to 0.05 and p-values are indicated where applicable.

### Data availability

Next Generation Sequencing data sets generated and analysed during the current study are available in the GEO repository under accession number GSEXXX.

### Code availability

The software used in this study is open source. Cellranger from 10X Genomics: https://support.10xgenomics.com/single-cell-gene-expression/software Seurat packages: https://cloud.r-project.org/web/packages/Seurat/index.html

### Results

### Cytokine profiling in MIS-C

To get an overview of immune pathways involved in MIS-C, we performed a screening of multiple cytokines and chemokines from patients during the acute phase of MIS-C and follow-up visits, and compared the results to paediatric controls 6 weeks post infection (p.i.) with SARS-CoV-2 (Figure 1a and Fig. 5). Patient characteristics of our cohort are comparable to those previously published(Carter, Fish et al. 2020, Consiglio, Cotugno et al. 2020, Porritt, Binek et al. 2021, Sacco, Castagnoli et al. 2022). We observed a similar unspecific cytokine profile as described previously(Carter, Fish et al. 2020, Consiglio, Cotugno et al. 2020, Gruber, Patel et al. 2020, Sacco, Castagnoli et al. 2022, Lee, Le Pen et al. 2023), with an upregulation of type 1- (Fig. 5a-b), type 2- (Fig. 5c-d), type 3- (Fig. 5e), and IL-1-cytokines (Fig. 5f), as well as various chemokines (Fig. 5g). As TGF-β is highly upregulated during severe COVID-19, we measured serum levels of TGF-β and compared them to our previously published TGF-β-data set from healthy young adults (less than 30 years old), flu-like illness (FLI) patients and SARS-CoV-2 infected adults with varying disease severity(Witkowski, Tizian et al. 2021) (Figure 1b). Strikingly, serum levels of TGF- β in MIS-C patients were as high (median: 398 pg/ml) as those found in severely affected COVID-19 patients (median: 415 pg/ml, p≥0.9999) and approximately 7-fold higher than in paediatric controls 6 weeks p.i. without MIS-C (median: 63 pg/ml p=0.0365), or in young healthy adults (median: 64 pg/ml, p=0.0072). Interestingly, there were a few paediatric patients with high TGF-β levels that did not develop MIS-C (two of which had an underlying rheumatic disease). TGF-β levels quickly dropped after start of treatment with immunoglobulins and methylprednisolone (Figure 1b), which was accompanied by a quick resolution of hyperinflammation. Of note, TGF-β levels showed the strongest negative correlation with time after start of therapy (Spearman r=-0.73, p=0.0003) of all measured cytokines (Figure 1b and Fig. 5h). This resembles our previous findings in severe COVID-19, where dexamethasone treatment correlated with decreased serum levels of TGF- β(Stefan Frischbutter 2023). For Kawasaki-Disease (KD), which closely resembles MIS-C, the genetic variants within the TGF-β-SMAD-pathway have been linked with genetic disease susceptibility(Shimizu, Jain et al. 2011, Cho, Han et al. 2014). Furthermore in KD, an upregulation of miR145 that mainly interacts with the TGF-β-pathway has been observed(Shimizu, Kim et al. 2013) and linked to disease pathology(Shimizu, Oharaseki et al. 2013). This highlights the role of TGF- β in hyperinflammatory syndromes.

In MIS-C, the upregulation of TGF-β can be directly linked to a previous infection with SARS-CoV-2, as the SARS-CoV-2 nucleoprotein interacts with SMAD3 and thereby activates the response to the TGF-β pathway(Wang, Chen et al. 2022), which was previously already described for the SARS-CoV nucleoprotein(Zhao, Nicholls et al. 2008). This SMAD3 activation subsequently also triggers upregulation of TGF-β, which is one of the TGF-β-response genes(Van Obberghen-Schilling, Roche et al. 1988). In addition, SARS-CoV-2 spike protein can interact with integrins to activate latent TGF-β(Biering, Gomes de Sousa et al. 2022). Recently, *TGFB1* (encoding TGF-β1) has been used in a 5-gene whole blood RNA expression signature to distinguish MIS-C from other inflammatory conditions(Jackson, Miglietta et al. 2023), hinting to a key biological function of TGF-β in MIS-C.

As TGF-β needs enzymatic cleavage to become biologically active(Taylor 2009, Worthington, Klementowicz et al. 2011), high serum levels alone do not necessarily correlate with biological activity. Therefore, we collected peripheral blood mononuclear cells (PBMC) from 11 representative patients during the acute phase of MIS-C and 4 otherwise healthy paediatric controls 6 weeks post-infection with SARS-CoV-2 who did not develop MIS-C (6w p.i. no MIS-C). We performed single-cell RNA sequencing (scRNAseq) of sorted activated T-cells (defined by expression of HLA-DR and CD38), memory B-cells, plasmablasts and monocytes (Fig. 6a-b). By separating the resulting uniform manifold approximation and projections (UMAP) by condition, we observed a distinct cluster of monocytes present only in controls and other clusters only in subgroups of patients (Figure 1c-d, Fig. 6c). Monocyte clusters 3 and 18 were defined by high expression of *CD163,* resembling monocytes found in patients with severe COVID-19(Schulte-Schrepping, Reusch et al. 2020, Wendisch, Dietrich et al. 2021). Others however, have shown that *CD163* is upregulated in monocytes by glucocorticoids(H6gger, Dreier et al. 1998, Schulz, Severin et al. 2019). To test if this observation was related to the hyperinflammation, or to the treatment patients were subjected to, we separated the UMAP so that each patient is depicted individually (Fig. 6c) and compared frequencies of cells annotated to each cluster, taking into account the treatment with different methylprednisolone doses (Figure 1d-f and Fig. 6d). Interestingly, the *CD163*^{high} monocyte clusters 3 and 18 were only detected in patients treated with methylprednisolone. To further prove that this effect can be attributed to treatment with glucocorticoids, we collected paired blood samples from 4 treatment-naive paediatric patients with newly diagnosed rheumatic diseases (2 patients with systemic lupus erythematosus, 1 patient with juvenile idiopathic arthritis and 1 patient with morphea) before and after a methylprednisolone pulse therapy (Fig. 7). We did not observe major differences in the activated T- or memory B-cell compartments, whilst observing the appearance of clusters containing *CD163* expressing monocytes only after methylprednisolone treatment (cluster 3 and 7). Thus, the effect seen on the monocyte clusters 3 and 18 can be attributed to the methylprednisolone treatment, whilst cluster 7 represents inflammation primed monocytes in MIS-C. Glucocorticoid treatment effects on activated T- and memory B-cells appears to be limited.

For MIS-C, the most prominent changes in the frequencies of enriched activated immune cells were observed within the activated T-cell compartment (Fig. 1e), with *Ki-67*⁺ proliferating T-cells (median 1.4% vs. 6.2%; p=0.0176) and activated cytotoxic T-cells (median 7.8% vs. 13.4%; p=0.0557) being especially enriched in MIS-C patients. This strong proliferation indicates recent activation of these T-cells via the T-cell receptor (TCR). Additionally, we observed decreased switched B-cells, and in two patients, an increase in B-cells expressing high levels of *CD83, CD69* and *TGFB1* (Fig. 1f). CD83 on B-cells has been shown to be upregulated by antigen-activated T-cells independently of their antigen specificity(Kretschmer, Kühl et al. 2011). Similar B-cells were previously found to be increased in patients with multiple sclerosis(Romme Christensen, Börnsen et al. 2013, McWilliam, Sellebjerg et al. 2018).

To determine whether induced TGF-β-levels are functionally active in the analysed immune cell subsets in MIS-C, we performed several single-cell Gene Set Enrichment Analysis (GSEA). For this, we analysed T-cells, B-cells and monocytes separately. Activated T-, B-cells and monocytes from MIS-C patients showed significant upregulation of the TGF-β induced gene sets using the hallmark genes set "TGF-β signalling"(Liberzon, Birger et al. 2015) (Fig. 8a-c). For T-cells, a previously described gene set was used in addition, which was generated from cytokine activated NK-cells in the presence or absence of TGF-β(Witkowski, Tizian et al. 2021). This further showed a strong enrichment of TGF-β induced genes in MIS-C (Fig. 2a, T cells: Normalised enrichment score (NES) median: 1.2 vs 1.4; p=1.304×10⁻¹⁶). Overall, this underlines the biological significance of the high TGF-β serum-levels observed. Further analysis of monocytes using the module "Li M200 Antigen processing and presentation" (Li, Rouphael et al. 2014) revealed a significant downregulation of genes involved in antigen presentation in the MIS-C monocytes (Fig. 2b, monocytes: 81% of cells with significant GSEA have negative NES in MIS-C vs. 18% in no-MIS-C; p<1×10⁻¹⁵). To a lesser extent, antigen processing and presentation genes were also downregulated in B cells (B-cells: 69% of cells with significant GSEA have negative NES in MIS-C vs. 55% in no-MIS-C; p=3.93×10⁻⁸) (Fig. 8d). Upregulation of NF-κB-signalling and a modulation of type I and type II IFN-signalling has been previously demonstrated in MIS-C monocytes(de Cevins, Luka et al. 2021). Likewise, we could demonstrate upregulation of these inflammatory pathways in MIS-C T- and B-cells (Fig. 8e-g). A strong upregulation of chemokines and their receptors (Fig. 8h-i) in MIS-C T- and B-cells and a downregulation (29% negative enriched in MIS-C vs 14% of controls; p=0.000183) in MIS-C monocytes, most prominently in the methylprednisolone primed *CD163*^{high} monocytes, was also observed (Fig. 8j). This was accompanied by a highly significant induction of the hallmark "TNF signalling via NF-κB" gene set (Fig. 8k-m), and "Interferon-a response" (Fig. 8n-p) and to a lesser extent also "Interferon-γ-response" (Fig. 8q-s). Overall, activated immune cells are simultaneously primed by different cytokines including a strong TGF-β signature.

### T-cell dysfunction in MIS-C is induced by TGF-β

We next wanted to determine the impact of this hyperinflammation on memory T-cell function. To quantify the reactivity of memory T-cells towards epitopes derived from previous viral infections in acute MIS-C and after recovery, we used a T-cell activation assay(Bacher, Schink et al. 2013), based on the rapid induction of CD69, CD154 and CD137 upon antigen specific stimulation(Frentsch, Arbach et al. 2005) (Fig. 2c-d, Fig. 9a). Interestingly, during acute MIS-C, both CD4⁺ and CD8⁺ T-cells co-cultured with antigen presenting cells and various viral peptides showed functional impairment by reduced expression of the early activation marker CD69 (Fig. 2d). Even specific reactivation by these viral peptides was impaired during the acute phase of MIS-C, ranging from 0.3-fold to 15-fold (median 1.6-fold, p=0.0396) for CD4⁺- and 0.04 to 10-fold (median 10-fold; p=0.0501) for CD8⁺ T-cells. Given the strong TGF-β instruction of T-cells in MIS-C (Fig. 2a) and because TGF-β has been implicated in terminating immune responses(Sanjabi, Oh et al. 2017), we investigated if the observed impairment in memory T-cell reactivation could be reproduced with T-cells derived from healthy donors when stimulated in the presence of TGF-β. Indeed, addition of TGF-β significantly impaired T-cell reactivity (Fig. 9b-c). Next, we investigated whether the observed impairment of memory T-cell reactivation could be effectively reversed by blocking antibodies against TGF-β1/2/3. To do so, we reactivated T-cells from healthy donors in media containing sera from MIS-C patients in the presence or absence of TGF-β1/2/3-blocking antibodies (Fig. 2e). For both CD4⁺ and CD8⁺ T-cells, blocking TGF-β1/2/3 increased T-cell overall reactivation, as frequencies of CD69⁺ memory T-cells increased (CD4⁺ T-cells: median 1.9-fold increase, p=0.0023; CD8⁺ T-cells: median 2.0-fold increase, p=0.0001). Additionally, specific reactivation was enhanced in CD4⁺ T-cells, indicated by increased frequencies of CD154⁺ CD69⁺ CD4⁺ memory T-cells (median 1.3-fold increase, p=0.014). We made a similar observation when using sera derived from severe COVID-19 patients (Fig 2f), suggesting that TGF-β induced immune malfunction is a hallmark of both MIS-C as well as severe COVID-19(Ferreira-Gomes, Kruglov et al. 2021, Witkowski, Tizian et al. 2021).

### TCR-repertoires indicate a marked EBV-linked inflammation in MIS-C

MIS-C is associated with oligoclonal expansion of T-cells with the variable Vβ TCR chain TCRVβ21.3(Moreews, Le Gouge et al. 2021, Porritt, Paschold et al. 2021, Sacco, Castagnoli et al. 2022, Lee, Le Pen et al. 2023). However as previously described(Moreews, Le Gouge et al. 2021, Porritt, Paschold et al. 2021, Lee, Le Pen et al. 2023), not all patients showed oligoclonal expansion of TCRVβ21.3+ T-cells (76% of CD4⁺ and 62% of CD8⁺ T-cells) when we assessed total PBMC by flow cytometry. (Fig. 10a). Surprisingly, this MIS-C-specific oligoclonal expansion was present in all patients in which we monitored TCRVβ21.3+ T-cells expansion after 1-3 days of high-dose treatment with methylprednisolone and intravenous immunoglobulins (Fig. 10a) (p=0.0499 for CD4⁺- and p=0.0372 CD8⁺-T-cells). Likewise, in the few patients where we measured TCRVβ21.3⁺ T-cells expansion consecutively during the acute phase, we observed an increase in oligoclonal expansion after the first few days of treatment and clinical improvement (data not shown). Initially, oligoclonal expansion of T-cells in MIS-C had been thought to be driven by the spike-protein acting as a superantigen(Porritt, Paschold et al. 2021, Hoste, Roels et al. 2022, Brodin 2023); however, increased expansion upon successful treatment (Fig. 10a), as well as further evidence reviewed by Jane Burns (Burns 2023) hints to a specific function of these T-cells. To identify which T-cells express *TRBV11-2* (encoding TCRVβ21.3), we analysed our scRNAseq data set. Interestingly, most TRBV11-2-positive T-cells mapped not only to the proliferating T-cell cluster 8, but also to the activated cytotoxic T-cell clusters 2 and 13 (Figure 3a), containing both CD8a⁺ and CD4⁺ T-cells (Figure 3b). Of note, independent of treatment, we observed oligoclonal expansion of *TRBV11-2*⁺ T-cells in all patients with MIS-C (Figure 3c). This indicates that by zooming in on the CD38⁺, HLA-DR^{high} T-cells, the relevant disease-driving cell population was analysed. Furthermore, this high-resolution analysis has the potential to be used as a diagnostic test for MIS-C in the future. Next, we verified the specificity of *TRBV11-2*⁺ T-cells by testing whether all, or only a subset of T-cells expressing *TRBV11-2,* were expanded in MIS-C. Hence, we looked at the *TRAV* genes (encoding the -cell receptor α-chain variable region) associated with *TRBV11-2* and identified a subset of full TCRs that could only be detected in MIS-C patients but not in the paediatric control group (Figure 3d, Fig. 10f). This further argues against a superantigenic expansion of *TRBV11-2*⁺ T-cells. We compared the HLA-haplotypes of our Berlin cohort to a previously published American cohort (Porritt, Paschold et al. 2021). Combining these two populations, we identified a predominance of *HLA-A*02* (28%, p≥0.9999),-*B*35* (24%, p=0.1056) and -C*04 (27%, p=0.7633), which was also prominently found in controls (Fig. 10c). However, we observed a significant predominance of the following HLA-class-II haplotypes: *HLA-DRB1*01* (0% vs. 19.4% p=0.0313) and *-DQB1*05* (0% vs. 23%, p=0.0027) (Fig. 10d-e). For ankylosing spondylitis (AS), an autoimmune disease with inflammation of joints and ligaments of the spine, it is known that specific HLA-haplotypes (namely *HLA-B*27)* combined with specific TCR repertoire *(TRBV1)* play a role in induction of inflammation, which is linked to specific peptides(May, Dulphy et al. 2002). Recently, for AS, a link between *HLA-8*27* and CD8 TCR clonotypes specific against EBV and CMV has been established(Hanson, Nel et al. 2020).

In severe SARS-CoV-2 infections, reactivation of herpesvirus has been observed in up to 82% of severe COVID-19 patients (Simonnet, Engelmann et al. 2021, Naendrup, Garcia Borrega et al. 2022, Chen, Ho et al. 2023). Additionally, CMV, EBV and human herpes virus 6 (HHV-6) reactivation is frequently observed in post-COVID-19-sequelae (Gold, Okyay et al. 2021, Saade, Moratelli et al. 2021, Zubchenko, Kril et al. 2022, Peluso, Deveau et al. 2023, Vojdani, Vojdani et al. 2023), with immune dysregulation and viral reactivation being a hypothesised mechanism for long-COVID (Davis, McCorkell et al. 2023). Immune profiling has identified specific immunological perturbations defining long-COVID, which include upregulation of TGF-β2 as well as different EBV epitopes (Klein, Wood et al. 2022).

Recently, a case-series of unexplained hepatitis in children, timely associated with the SARS-CoV-2 Omicron outbreak, was linked to a specific AdV- strain (Morfopoulou, Buddle et al. 2023). The affected children had a high prevalence for prior infection with SARS-CoV-2 (UKHSA 2022, Morfopoulou, Buddle et al. 2023). Given the observed immune dysfunction in MIS-C and the potential association of viral hepatitis cases with post-SARS-CoV-2 immune dysfunction, we subsequently investigated whether there was a simultaneous reactivation of viruses in MIS-C, which could potentially explain the systemic hyperinflammation. Therefore, we compared the oligoclonal expanded TCRs of MIS-C with a self-generated atlas of virus-specific TCRs, focusing on EBV and CMV. For this, we isolated EBV-, CMV-, adenovirus (AdV)-, and SARS-CoV-2- virus-specific TCRs from healthy donors using the antigen-reactive T-cell enrichment (ARTE) assay (Bacher, Schink et al. 2013)(Figure 3e), generated TCR libraries and combined the obtained data with our previously published SARS-CoV-2- and measles, mumps and rubella (MMR)-specific TCR repertoire dataset (Ferreira-Gomes, Kruglov et al. 2021). Frequencies of specific TCR-alpha chains in combination with TRBV11-2 were clustered in an unbiased manner. This resulted in combined clustering of all MIS-C TCR repertoires together with EBV-specific TCR repertoires from 4 out of 5 donors (Figure 3f), suggesting that the oligoclonal expanded MIS-C *TRBV11-2*⁺ T-cells could be EBV specific. To further test this hypothesis, we analysed if *TRBV11-2*⁺ T-cells are involved in controlling EBV reactivation. We isolated T-cells from healthy donors and enriched both TCRVβ21.3+ CD8⁺ and CD4⁺ T-cells as some CD4⁺ T-cells also clustered in the cytotoxic T-cell cluster (Figure 3b). As a control, we used the T-cells depleted of cells expressing TCRVβ21.3. After polyclonal expansion, T-cells were co-cultured with EBV-transformed B-cells (lymphoblastic cell line, LCL) derived from the same donor. TCRVβ21.3+ CD8⁺ T-cells showed an increased killing capacity (16.5 median percentage-points increase, p=0.0156) of these HLA identical EBV-infected B-cells (Figure 3h). This coincided with a 2.5-fold increased frequency of CD107a⁺ T-cells (p=0.0313), indicating specific killing of infected B-cells by degranulation(Betts, Brenchley et al. 2003). As we only detected a small fraction of CD4⁺ cytotoxic T-cells, and to allow for upregulation of the cytotoxic phenotype during co-culturing, CD4⁺ TCRVβ21.3+ T-cells were cultured in a ratio of 30 T-cells to 1 LCL for 24 hours. As observed for CD8⁺ T-cells, CD4⁺ TCRVβ21.3+ T-cells had a median enhanced killing capability of 14.5 percentage-points for LCL (p=0.0273) (Figure 3i). Collectively, these results suggest an oligoclonal expansion of dysfunctional EBV-specific cytotoxic T-cells. In light of our previous findings, these T-cells, despite being fully armed, seem incapable of efficiently attaching to and killing EBV-infected cells, as it has been shown for TGF-β- instructed NK-cells which are_incapable of attaching to SARS-CoV-2-infected cell (Witkowski, Tizian et al. 2021).

### TGF-β induced virus reactivation in MIS-C

To test if EBV-reactivation is common in MIS-C, we checked if any of our cohort had been screened by PCR for EBV virus load quantification while in hospital. Although only anecdotal as only a few patients had been tested, we found EBV and HHV-6 DNA in a myocardial biopsy. To this end, we looked for signs of EBV-reactivation in an independent MIS-C cohort from Lyon with higher rates of testing for virus reactivation. Combined in both cohorts, 26% of MIS-C patients were routinely tested for EBV reactivation/infection by PCR. Of the 23 patients tested, EBV viremia in peripheral blood could be detected by PCR in 10 patients. This led us to assess the prevalence of antibodies against EBV and other common infection-causing latent viruses in our combined MIS-C cohort. Since all patients were treated with intravenous immunoglobulins (IVIG) consisting of highly purified human IgG, only samples collected before initiation of IVIG treatment were taken into consideration to complement the routinely tested serology (Fig. 11a). 71 % of the tested patients in our combined cohort were seropositive compared to 56% (p=0.0447) of a local age-matched pre-pandemic paediatric control (Beer 2017). By detection of virus-specific IgM, we found a high incidence of latent virus reactivation at the serological level (Fig. 11b). Both EBV and CMV, but not HHV-6 or HSV-1, seroprevalences were markedly increased when compared to an age-matched epidemiological seroprevalence (Enders, Biber et al. 1990, Sauerbrei, Schmitt et al. 2011, Beer 2017, Hoehl, Berger et al. 2020)(Figure 4a, Fig. 11c-e). To find further evidence of EBV-reactivation in SARS-CoV-2-sequelae, we looked for EBV envelope mRNA, a direct sign of lytic EBV replication, in our previously published severe COVID-19 plasmablast dataset (Ferreira-Gomes, Kruglov et al. 2021). Indeed, EBV envelope mRNA was found in 10 out of the 12 analysed patients (p=0.0008), in roughly in 1 out of 2000 plasmablasts. These findings prompted us to sequence plasmablasts from 3 MIS-C patients. Among these 3 patients, we found EBV envelope mRNA in 2 of them, whilst we could not detect it in any of the 6 healthy controls or mild COVID-19 individuals analysed (p=0.023). As viral infections can trigger EBV-reactivation, we tested if TGF-β is responsible for the prolonged viral reactivation. In line with previously published data(Liang, Chen et al. 2002), TGF-β induced the lytic cycle (quantified by the expression-levels of the early transcription factor of the lytic cycle *BZFL1 (Liang, Chen et al.* 2002)) of EBV-replication in LCL (Figure 4b). Furthermore, in LCL treated with sera from MIS-C or severe COVID-19 patients, a prior neutralization of TGF-β reduced the induction of the lytic cycle. Thus, the high TGF-β levels in MIS-C and severe COVID-19 patients increased susceptibility to EBV-reactivation.

Taken together, our data shows a role of SARS-CoV-2 induced upregulation of TGF-β in the pathogenesis of MIS-C. Our findings demonstrate that elevated levels of TGF-β lead to immune dysfunction and limit T-cell surveillance of EBV-infected B-cells. These mechanisms collectively contribute to viral-sepsis-induced hyperinflammation. Therefore, targeted intervention involving TGF-β blockade is a new approach for the management of MIS-C and also other inflammatory (post-)COVID-19 sequelae. In specific cases, beyond (post-)COVID-19 sequelae, therapies such as the administration of autologous EBV or SARS-CoV-2-specific T-cells engineered to be non-sensitive to TGF-β or immunosuppression (Peter, Wendering et al. 2022) can alleviate undesirable virus-induced hyperinflammation.

### REFERENCES

Altschul, S. F., et al. (1990). "Basic local alignment search tool." J Mol Biol 215(3): 403-410.
Bacher, P., et al. (2013). "Antigen-Reactive T Cell Enrichment for Direct, High-Resolution Analysis of the Human Naive and Memory Th Cell Repertoire." The Journal of Immunology 190(8): 3967-3976.
Beer, A. D. (2017). Die Prävalenz des Epstein-Barr-Virus im Stadt-Land-Gefälle bei Kindern und Jugendlichen bis zum vollendeten 16. Lebensjahr. Charité Universitätsmedizin Berlin. REFUBIUM - REPOSITORIUM DER FREIEN UNIVERSITAT BERLIN, Charité Universitätsmedizin Berlin. MD: 78.
Belot, A. and D. Levy-Bruhl (2020). "Multisystem Inflammatory Syndrome in Children in the United States." N Engl J Med 383(18): 1793-1794.
Betts, M. R., et al. (2003). "Sensitive and viable identification of antigen-specific CD8+ T cells by a flow cytometric assay for degranulation." J Immunol Methods 281(1-2): 65-78.
Biering, S. B., et al. (2022). "SARS-CoV-2 Spike triggers barrier dysfunction and vascular leak via integrins and TGF-β signaling." Nat Commun 13(1): 7630.
Brodin, P. (2023). "Exaggerated responses to a virus long gone." Science 379(6632): 538-539. Multisystem inflammatory syndrome in children is caused by abnormal cell activation.
Burns, J. C. (2023). "MIS-C: myths have been debunked, but mysteries remain." Nat Rev Rheumatol 19(2): 70-71.
Carter, M. J., et al. (2020). "Peripheral immunophenotypes in children with multisystem inflammatory syndrome associated with SARS-CoV-2 infection." Nature Medicine 26(11): 1701-1707.
CDC. Retrieved 06.10.2022, from Multisystem Inflammatory Syndrome in Children (MIS-C) Associated with Coronavirus Disease 2019 (COVID-19).
Chen, Y. C., et al. (2023). "Long-term risk of herpes zoster following COVID-19: A retrospective cohort study of 2 442 686 patients." J Med Virol 95(4): e28745.
Cheng, M. H., et al. (2020). "Superantigenic character of an insert unique to SARS-CoV-2 spike supported by skewed TCR repertoire in patients with hyperinflammation." Proc Natl Acad Sci U S A 117(41): 25254-25262.
Cho, J. H., et al. (2014). "Genetic polymorphism of SMAD5 is associated with Kawasaki disease." Pediatr Cardiol 35(4): 601-607.
Consiglio, C. R., et al. (2020). "The Immunology of Multisystem Inflammatory Syndrome in Children with COVID-19." Cell 183(4): 968-981.e967.
Davis, H. E., et al. (2023). "Long COVID: major findings, mechanisms and recommendations." Nat Rev Microbiol 21(3): 133-146.
de Cevins, C., et al. (2021). "A monocyte/dendritic cell molecular signature of SARS-CoV-2-related multisystem inflammatory syndrome in children with severe myocarditis." Med 2(9): 1072-1092.e1077.
Enders, G., et al. (1990). "Prevalence of antibodies to human herpesvirus 6 in different age groups, in children with exanthema subitum, other acute exanthematous childhood diseases, Kawasaki syndrome, and acute infections with other herpesviruses and HIV." Infection 18(1): 12-15.
Ferreira-Gomes, M., et al. (2021). "SARS-CoV-2 in severe COVID-19 induces a TGF-β-dominated chronic immune response that does not target itself." Nat Commun 12(1): 1961.
Frentsch, M., et al. (2005). "Direct access to CD4+ T cells specific for defined antigens according to CD154 expression." Nat Med 11(10): 1118-1124.
Frischbutter S, Durek P, Witkowski M, Angermair S, Treskatsch S, Maurer M, Radbruch A, Mashreghi MF. Serum TGF-β as a predictive biomarker for severe disease and fatality of COVID-19. Eur J Immunol. 2023 Jun 30:e2350433. doi: 10.1002/eji.202350433. Epub ahead of print. PMID: 37386908.
Gold, J. E., et al. (2021). "Investigation of Long COVID Prevalence and Its Relationship to Epstein-Barr Virus Reactivation." Pathogens 10(6).
Gruber, C. N., et al. (2020). "Mapping Systemic Inflammation and Antibody Responses in Multisystem Inflammatory Syndrome in Children (MIS-C)." Cell 183(4): 982-995.e914.
Hanson, A. L., et al. (2020). "Altered Repertoire Diversity and Disease-Associated Clonal Expansions Revealed by T Cell Receptor Immunosequencing in Ankylosing Spondylitis Patients." Arthritis Rheumatol 72(8): 1289-1302.
Hao, Y., et al. (2021). "Integrated analysis of multimodal single-cell data." Cell 184(13): 3573-3587.e3529.
Hoehl, S., et al. (2020). "Thirty years of CMV seroprevalence-a longitudinal analysis in a German university hospital." Eur J Clin Microbiol Infect Dis 39(6): 1095-1102.
Högger, P., et al. (1998). "Identification of the integral membrane protein RM3/1 on human monocytes as a glucocorticoid-inducible member of the scavenger receptor cysteine-rich family (CD163)." J Immunol 161(4): 1883-1890.
Hoste, L., et al. (2021). "TIM3+TRBV11-2 T cells and IFNy signature in patrolling monocytes and CD16+ NK cells delineate MIS-C." Journal of Experimental Medicine 219(2).
Hoste, L., et al. (2022). "TIM3+ TRBV11-2 T cells and IFNy signature in patrolling monocytes and CD16+ NK cells delineate MIS-C." J Exp Med 219(2).
Jackson, H. R., et al. (2023). "Diagnosis of multisystem inflammatory syndrome in children by a whole-blood transcriptional signature." Journal of the Pediatric Infectious Diseases Society.
Klein, J., et al. (2022). "Distinguishing features of Long COVID identified through immune profiling." medRxiv.
Kretschmer, B., et al. (2011). "Activated T cells induce rapid CD83 expression on B cells by engagement of CD40." Immunol Lett 136(2): 221-227.
Lambert, G. P. (2004). "Role of Gastrointestinal Permeability in Exertional Heatstroke." Exercise and Sport Sciences Reviews 32(4).
Lee, D., et al. (2023). "Inborn errors of OAS-RNase L in SARS-CoV-2-related multisystem inflammatory syndrome in children." Science 379(6632): eabo3627.
Li, S., et al. (2014). "Molecular signatures of antibody responses derived from a systems biology study of five human vaccines." Nat Immunol 15(2): 195-204.
Liang, C. L., et al. (2002). "Epstein-Barr virus BZLF1 gene is activated by transforming growth factor-beta through cooperativity of Smads and c-Jun/c-Fos proteins." J Biol Chem 277(26): 23345-23357.
Liberzon, A., et al. (2015). "The Molecular Signatures Database (MSigDB) hallmark gene set collection." Cell Syst 1(6): 417-425.
Loomba, R. S., et al. (2020). "COVID-19 and Hyperinflammatory Syndrome in Children: Kawasaki Disease with Macrophage Activation Syndrome in Disguise?" Cureus 12(8): e9515.
Lun, A. T. L., et al. (2019). "EmptyDrops: distinguishing cells from empty droplets in droplet-based single-cell RNA sequencing data." Genome Biology 20(1): 63.
May, E., et al. (2002). "Conserved TCR beta chain usage in reactive arthritis; evidence for selection by a putative HLA-B27-associated autoantigen." Tissue Antigens 60(4): 299-308.
McWilliam, O., et al. (2018). "B cells from patients with multiple sclerosis have a pathogenic phenotype and increased LTα and TGFβ1 response." J Neuroimmunol 324: 157-164.
Moosmann, A., et al. (2002). "B cells immortalized by a mini-Epstein-Barr virus encoding a foreign antigen efficiently reactivate specific cytotoxic T cells." Blood 100(5): 1755-1764.
Mootha, V. K., et al. (2003). "PGC-1alpha-responsive genes involved in oxidative phosphorylation are coordinately downregulated in human diabetes." Nat Genet 34(3): 267-273.
Moreews, M., et al. (2021). "Polyclonal expansion of TCR Vbeta 21.3(+) CD4(+) and CD8(+) T cells is a hallmark of Multisystem Inflammatory Syndrome in Children." Sci Immunol 6(59).
Morfopoulou, S., et al. (2023). "Genomic investigations of unexplained acute hepatitis in children." Nature 617(7961): 564-573.
Naendrup, J. H., et al. (2022). "Reactivation of EBV and CMV in Severe COVID-19-Epiphenomena or Trigger of Hyperinflammation in Need of Treatment? A Large Case Series of Critically ill Patients." J Intensive Care Med 37(9): 1152-1158.
Peluso, M. J., et al. (2023). "Chronic viral coinfections differentially affect the likelihood of developing long COVID." J Clin Invest 133(3).
Penner, J., et al. (2021). "6-month multidisciplinary follow-up and outcomes of patients with paediatric inflammatory multisystem syndrome (PIMS-TS) at a UK tertiary paediatric hospital: a retrospective cohort study." Lancet Child Adolesc Health 5(7): 473-482.
Peter, L., et al. (2022). "Tacrolimus-resistant SARS-CoV-2-specific T cell products to prevent and treat severe COVID-19 in immunosuppressed patients." Mol Ther Methods Clin Dev 25: 52-73.
Pfeifer, J., et al. (2022). "Autoantibodies against interleukin-1 receptor antagonist in multisystem inflammatory syndrome in children: a multicentre, retrospective, cohort study." Lancet Rheumatol 4(5): e329-e337.
Porritt, R. A., et al. (2021). "The autoimmune signature of hyperinflammatory multisystem inflammatory syndrome in children." The Journal of Clinical Investigation 131(20).
Porritt, R. A., et al. (2021). "HLA class I-associated expansion of TRBV11-2 T cells in multisystem inflammatory syndrome in children." J Clin Invest 131(10).
RCPCH (2020). Retrieved 06.10.2022, from https://www.rcpch.ac.uk/sites/default/files/2020-05/COVID-19-Paediatric-multisystem-%20inflammatory%20syndrome-20200501 .pdf.
Riphagen, S., et al. (2020). "Hyperinflammatory shock in children during COVID-19 pandemic." The Lancet 395(10237): 1607-1608.
Romme Christensen, J., et al. (2013). "Systemic inflammation in progressive multiple sclerosis involves follicular T-helper, Th17- and activated B-cells and correlates with progression." PLoS One 8(3): e57820.
Saade, A., et al. (2021). "Herpesvirus reactivation during severe COVID-19 and high rate of immune defect." Infect Dis Now 51(8): 676-679.
Sacco, K., et al. (2022). "Immunopathological signatures in multisystem inflammatory syndrome in children and pediatric COVID-19." Nat Med 28(5): 1050-1062.
Sanjabi, S., et al. (2017). "Regulation of the Immune Response by TGF-β: From Conception to Autoimmunity and Infection." Cold Spring Harb Perspect Biol 9(6).
Sauerbrei, A., et al. (2011). "Seroprevalence of herpes simplex virus type 1 and type 2 in Thuringia, Germany, 1999 to 2006." Euro Surveill 16(44).
Sayers, E. W., et al. (2022). "Database resources of the national center for biotechnology information." Nucleic Acids Res 50(D1): D20-d26.
Schulte-Schrepping, J., et al. (2020). "Severe COVID-19 Is Marked by a Dysregulated Myeloid Cell Compartment." Cell 182(6): 1419-1440.e1423.
Schulz, D., et al. (2019). "In-Depth Characterization of Monocyte-Derived Macrophages using a Mass Cytometry-Based Phagocytosis Assay." Sci Rep 9(1): 1925.
Shimizu, C., et al. (2011). "Transforming growth factor-beta signaling pathway in patients with Kawasaki disease." Circ Cardiovasc Genet 4(1): 16-25.
Shimizu, C., et al. (2013). "Differential expression of miR-145 in children with Kawasaki disease." PLoS One 8(3): e58159.
Shimizu, C., et al. (2013). "The role of TGF-β and myofibroblasts in the arteritis of Kawasaki disease." Hum Pathol 44(2): 189-198.
Simonnet, A., et al. (2021). "High incidence of Epstein-Barr virus, cytomegalovirus, and human-herpes virus-6 reactivations in critically ill patients with COVID-19." Infect Dis Now 51(3): 296-299.
Stefan Frischbutter, P. D., Mario Witkowski, Stefan Angermair, Sascha Treskatsch, Marcus Maurer, Andreas Radbruch, Mir-Farzin Mashreghi (2023). "Serum TGFβ as a predictive biomarker for severe disease and fatality of COVID-19 " European Journal of Immunology.
Subramanian, A., et al. (2005). "Gene set enrichment analysis: a knowledge-based approach for interpreting genome-wide expression profiles." Proc Natl Acad Sci U S A 102(43): 15545-15550.
Taylor, A. W. (2009). "Review of the activation of TGF-beta in immunity." J Leukoc Biol 85(1): 29-33.
UKHSA (2022). "Investigation into acute hepatitis of unknown aetiology in children in England: technical briefing 4 ". Retrieved 21.05.2023, 2023.
Van Obberghen-Schilling, E., et al. (1988). "Transforming growth factor beta 1 positively regulates its own expression in normal and transformed cells." J Biol Chem 263(16): 7741-7746.
Verdoni, L., et al. (2020). "An outbreak of severe Kawasaki-like disease at the Italian epicentre of the SARS-CoV-2 epidemic: an observational cohort study." The Lancet 395(10239): 1771-1778.
Vojdani, A., et al. (2023). "Persistent SARS-CoV-2 Infection, EBV, HHV-6 and Other Factors May Contribute to Inflammation and Autoimmunity in Long COVID." Viruses 15(2).
Wang, W., et al. (2022). "SARS-CoV-2 N Protein Induces Acute Kidney Injury via Smad3-Dependent G1 Cell Cycle Arrest Mechanism." Adv Sci (Weinh) 9(3): e2103248.
Wendisch, D., et al. (2021). "SARS-CoV-2 infection triggers profibrotic macrophage responses and lung fibrosis." Cell 184(26): 6243-6261.e6227.
Witkowski, M., et al. (2021). "Untimely TGFβ responses in COVID-19 limit antiviral functions of NK cells." Nature 600(7888): 295-301.
Worthington, J. J., et al. (2011). "TGFβ: a sleeping giant awoken by integrins." Trends Biochem Sci 36(1): 47-54.
Yonker, L. M., et al. (2021). "Multisystem inflammatory syndrome in children is driven by zonulin-dependent loss of gut mucosal barrier." The Journal of Clinical Investigation 131(14).
Zhao, X., et al. (2008). "Severe acute respiratory syndrome-associated coronavirus nucleocapsid protein interacts with Smad3 and modulates transforming growth factor-beta signaling." J Biol Chem 283(6): 3272-3280.
Zhu, L., et al. (2020). "Clinical characteristics of a case series of children with coronavirus disease 2019." Pediatric Pulmonology 55(6): 1430-1432.
Zubchenko, S., et al. (2022). "Herpesvirus infections and post-COVID-19 manifestations: a pilot observational study." Rheumatol Int 42(9): 1523-1530.

## Claims

1. A TGF-β inhibitor for use in the treatment of virus-induced hyperinflammation or (post-) COVID-19 symptoms or sequelae, wherein the patient is suffering from or experiencing elevated (above-normal) expression, excretion and/or blood levels of transforming growth factor-β (TGF-β).

2. The TGF-β inhibitor for use according to claim 1, wherein the virus-induced hyperinflammation is associated with, or induced by a COVID-19 infection.

3. The TGF-β inhibitor for use according to any one of claims 1 or 2, wherein the virus-induced hyperinflammation is a severe acute hyperinflammatory shock or a multisystem inflammatory syndrome.

4. The TGF-β inhibitor for use according to any one of the preceding claims, wherein the sequelae of COVID-19 comprise the reactivation of viruses in the subject, preferably the reactivation of human Herpesviridae (HHV1-8).

5. The TGF-β inhibitor for use according to any one of claims 2-4, wherein the COVID-19-induced hyperinflammation is a multisystem inflammatory syndrome in children (MIS-C).

6. The TGF-β inhibitor for use according to any one of claims 2-5, wherein the patient suffering from COVID-19 induced hyperinflammation is a child, preferably wherein the patient is younger than 21 years of age, more preferably younger than 16 years of age.

7. The TGF-β inhibitor for use according to any one of the preceding claims, wherein the (post-)COVID-19 symptoms or sequelae are Long-COVID-19, (post-)COVID-19-infection symptoms and/or the reactivation of Herpesviridae in the subject.

8. The TGF-β inhibitor for use according to any one of the preceding claims, wherein the TGF-β inhibitor is a chemical or pharmaceutical compound, preferably a small molecule compound.

9. The TGF-β inhibitor for use according to any of the preceding claims, wherein the TGF- β inhibitor is an inhibitor of a transforming growth factor-β receptor (TGF-βR), a SMAD inhibitor and/or an inhibitor of MAP-kinases.

10. The TGF-β inhibitor for use according to any of the preceding claims, wherein the TGF-β inhibitor is an antibody against TGF-β-1 and/or TGF-β-2 and/or TGF-β-3.

11. A TGF-β inhibitor for use in the treatment of the reactivation of a virus in a patient, wherein the patient is suffering from or experiencing elevated (above-normal) expression, excretion and/or blood levels of transforming growth factor-β (TGF-beta).

12. The TGF-β inhibitor for use according to the preceding claim, wherein the reactivation of the virus in a patient is associated with or induced by a COVID-19 infection.

13. The TGF-β inhibitor for use according to the preceding claim, wherein the virus is a human herpes virus (Herpesviridae / HHV1-8).

14. A T-cell for the use in the treatment of virus reactivation and/or SARS-CoV-2-induced hyperinflammation, (long-term) symptoms and/or sequelae, wherein the T-cell is treated and/or genetically engineered to be non-sensitive to TGF-β and/or immunosuppression, and wherein the T cell preferably expresses CD4 and/or CD8.

15. A method for stimulating T cells comprising the steps of
a. Providing a population of T cells,
b. Optionally incubating the T cells with a TGF- β inhibitor and/or genetically engineering the T cells to be resistant or insensitive to TGF-β stimulation,
c. Providing a population of antigen-presenting cells,
d. Incubating the population of antigen-presenting cells with viral particles or parts thereof,
e. Stimulating the population of T cells, comprising co-culturing the population of T cells with the population of antigen-presenting cells provided in step c.
